# EUROPÄISCHE PATENTSCHRIFT

(11) **EP 1 391 457 B1**
(45) Veröffentlichungstag und Bekanntmachung des Hinweises auf die Patenterteilung: **25.12.2013**
(21) Anmeldenummer: 03024899.1
(22) Anmeldetag: 28.10.1999
(51) Int. Cl.: C07D 235/18, C07D 403/10, C07D 401/10, A61K 31/4184, A61K 31/454, A61K 31/551, A61P 9/00

(54) **Substituierte 2-Phenylbenzimidazole und deren Verwendung als PARP Inhibitoren**
Substituted 2-Phenylbenzimidazoles and their use as PARP inhibitors
2-Phénylbenzimidazoles substitués et leur utilisation comme inhibiteurs de PARP

(30) Priorität: 03.11.1998 DE 19850709; 16.11.1998 DE 19852801; 01.03.1999 DE 19908733
(43) Veröffentlichungstag der Anmeldung: 25.02.2004
(62) Teilanmeldung aus: 99955894.3
(73) Patentinhaber: AbbVie Deutschland GmbH & Co KG, 65205 Wiesbaden (DE)
(72) Erfinder: Lubisch, Wilfried, 69115 Heidelberg (DE); Kock, Michael, 67105 Schifferstadt (DE); Höger, Thomas, Dr., 69514 Laudenbach (DE)
(74) Vertreter: Grünecker, Kinkeldey, Stockmair & Schwanhäusser

(56) Entgegenhaltungen:
- WO-A-97/04771
- GRIFFIN R J ET AL: "Novel benzimidazole and quinazolinone inhibitors of the DNA repair enzyme poly(ADP-ribose)polymerase" PHARMACEUTICAL SCIENCES, Bd. 2, Nr. 1, 1996, Seiten 43-47, XP000886156

## Beschreibung

Die vorliegende Erfindung betrifft neuartige 2-Phenylbenzimidazole, ihre Herstellung mit neuen Zwischenprodukten und die Verwendung als Inhibitoren des Enzyms Poly(ADP-ribose)polymerase oder PARP (EC 2.4.2.30) zur Herstellung von Arzneimitteln.

Poly(ADP-ribose)polymerase (PARP), bzw. wie es auch genannt wird Poly(ADP-ribose)synthase (PARS), stellt ein regulatorisches Enzym dar, das in Zellkernen gefunden wird (K. Ikai et al., J. Histochem. Cytochem. 1983, 31, 1261-1264). Man nimmt an, daß PARP eine Rolle bei der Reparatur von DNA-Brüchen spielt (M.S. Satoh et al., Nature 1992, 356, 356-358). Schädigungen oder Brüche der DNA-Stränge aktivieren das Enzym PARP, das, wenn es aktiviert ist, die Übertragung von ADP-Ribose aus NAD katalysiert (S. Shaw, Adv. Radiat. Biol., 1984, 11, 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei einer Reihe von pathophysiologischen Zuständen beobachtet und man geht davon aus, daß diese Anhäufung von Radikalen zu den beobachteten Zell- bzw. Organschäden führen oder beitragen. Dazu zählt von zum Beispiel ischämische Zustände von Organen wie im Schlaganfall, Herzinfarkt (C. Thiemermann et al., Proc. Natl. Acad. Sci. USA, 1997, 94, 679-683) oder Ischämie der Nieren, aber auch Reperfusionsschäden wie sie zum Beispiel nach der Lyse von Herzinfarkt auftreten (s. oben: C. Thiemermann et al.). Die Hemmung von dem Enzym PARP könnte demzufolge ein Mittel sein, um diese Schäden mindestens zum Teil zu verhindern oder abzumildern. PARP-Inhibitoren könnten somit ein neues Therapieprinzip zur Behandlung von einer Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. Cancer Chemo. Pharmacol. 1988, 22, 303).

Nicht limitierende Beispiele für Tumoren sind Leukämie, Glioblastome, Lymphome, Melanome, Mama- und Cervicalkarzinome.

Zudem wurde gefunden, daß PARP-Inhibitoren immunosuppressive Wirkung zeigen können (D. Weltin et al. Int. J. Immunopharmacol. 1995, 17, 265-271).

Es wurde ebenfalls entdeckt, daß PARP bei immunologischen Erkrankungen bzw. Krankheiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. Infammation 1996, 20, 203-215; W. Ehrlich et al. Rheumatol. Int. 1995, 15, 171-172; C. Szabo et al., Proc. Natl. Acad. Sci. USA 1998, 95, 3867-3872; S. Cuzzocrea et al. Eur. J. Pharmacol. 1998, 342, 67-76).

Unter PARP im Sinne dieser Erfindung werden auch Isoenzyme des oben beschriebenen PARP-Enzyms verstanden. Solche Isoenzyme sind z.B. PARP II und PARP III.

Weiterhin zeigte der PARP-Inhibitor 3-Aminobenzamid protektive Effekte in einem Model für den Kreislaufschock (S. Cuzzocrea et al., Br. J. Pharmacol. 1997, 121, 1065-1074).

2-Phenylbenzimidazole sind vielfach beschrieben worden. So sind in DE 38 30 060 alkylierte Derivate als Inhibitoren der Erythrozytenaggregation offengelegt. In DE 35 22 230 ist ein Ester-Derivat vom 2-Phenylbenzimidazol als Inhibitor der Plättchenaggragation aufgeführt. Halogen-substituierte 2-Phenylbenzimizaole, die am Phenyl-Ring substituierte Amin-Reste tragen, sind in WO 98/06703 als MCP-1-Antagonisten beschrieben worden.

Ebenfalls sind 2-Phenyl-benzimidazole bekannt, bei denen die Benzimidazol-Gruppe durch eine Amid-Gruppe substituiert ist. 5-Amido-Derivate des 2-Phenylbenzimidazols, die am Phenyl-Ring Alkyloxy-Reste tragen, sind in WO 94/12461 als Inhibitoren der cAMP-Phosphodiesterase beschrieben worden. Für analoge Derivate wurde in DE 35 46 575 (z.B. Beispiel 15) gefunden, daß diese Verbindungen positiv inotrope Effekte auslösen. Ebenfalls 4-Amido-Derivate, die in 3-Stellung einen Pyridyl-Rest tragen, sind in WO 97/48697 als Inhibitoren der cAMP-Phosphodiesterase aufgeführt.

Die Synthese von 2-Phenyl-benzimidazyl-4-amiden ist in J. Chem. Soc. Perkin Trans 1, 1979, 2303-2307, beschrieben worden. Analoge Verbindungen, die am Amid-Rest noch eine substituierte AlkylKette tragen, und die cytotoxische Wirkung haben sollen, sind in J. Med. Chem. 1990, 33, 814-819, aufgeführt. In WO 97/04771 und in Pharm. Sci. 1996, 2, 43-47, sind dagegen Benzimidazol-4-amide aufgeführt, die das PARS hemmen. Insbesondere sind in WO 97/04771 Derivate als wirksam beschrieben, die einen Phenyl-Ring in 2-Stellung tragen, wobei der Phenyl-Ring noch mit einfachen Substituenten, wie Nitro, Methoxy und CF3, substituiert sein kann. Obwohl diese Substanzen zum Teil gute Hemmung des Enzyms PARP zeigen, haben die dort beschriebenen Derivate den Nachteil, daß sie nur wenig oder keine Löslichkeit in wäßrigen Lösungen zeigen und somit nicht als wäßrige Lösung appliziert werden können.

In einer Reihe von Therapien wie Schlaganfall werden die Wirkstoffe intravenös als Infusionslösung appliziert. Dazu ist es notwendig, Substanzen, hier PARP-Inhibitoren, zur Verfügung zu haben, die ausreichende Wasserlöslichkeit bei physiologischen pH-Werten oder angenäherten pH-Werten (z.B: pH-Werten von 5 bis 8) aufweisen, so daß eine Infusionslösung hergestellt werden kann. Viele der beschriebenen PARP-Inhibitoren, insbesondere die besser wirksamen PARP-Inhibitoren, haben jedoch den Nachteil, daß sie nur geringe oder keine Wasserlöslichkeit bei diesen pH-Werten zeigen und somit nicht für eine intravenöse Applikation in Frage kommen. Derartige Wirkstoffe können nur mit Hilfsstoffen, die die Wasserlöslichkeit vermitteln sollen, appliziert werden (vgl. WO 97/04771). Diese Hilfsstoffe, zum Beispiel Polyethylenglykol und Dimethysulfoxid, verursachen häufig Nebeneffekte oder sind sogar unverträglich. Es ist Aufgabe der vorliegenden Erfindung gut wirksame PARP-Inhibitoren mit ausreichender Wasserlöslichkeit zu erhalten.

Es wurde überraschenderweise gefunden, daß 2-Phenyl-benzimidazole, die am Phenyl-Ring mit Alkoxy-Resten substituiert sind und an der Alkoxy-Seitenkette noch einen Amin-Rest tragen, gut wirksame Inhibitoren darstellen, die aber durch den Einbau des aliphatischen Amin-Restes eine Salzbildung mit Säuren ermöglichen und dadurch eine deutlich verbesserte Wasserlöslichkeit zeigen.

Das Ziel der vorliegenden Erfindung wurde erreicht durch neue 2-Phenylbenzimidazol-Derivate der allgemeinen Formel I, die gegenüber den bereits beschriebenen Verbindungen Vorteile zeigen und potente PARP-Inhibitoren darstellen und zugleich auch ausreichende Wasserlöslichkeit zeigen, die eine Applikation als Infusionslösung ermöglicht.

Gegenstand der vorliegenden Erfindung sind substituierte 2-Phenylbenzimidazole der allgemeinen Formel I oder II 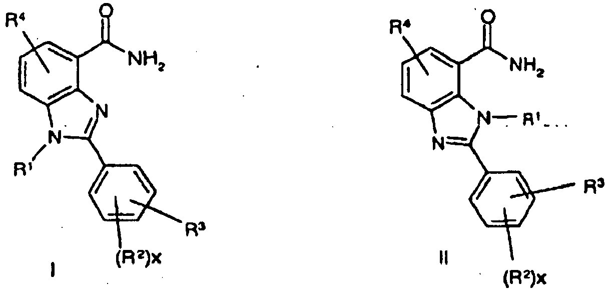 worin
- R¹: Wasserstoff, verzweigtes und unverzweigtes C₁-C₆-Alkyl, wobei ein C-Atom des Alkyl-Restes noch OR¹¹ oder eine Gruppe R⁵ tragen kann, wobei
- R¹¹: Wasserstoff oder C₁-C₄-Alkyl bedeutet, und
- R²: Wasserstoff, Chlor, Brom, Jod, Fluor, CF3, Nitro, NHCOR²¹, NR²²R²³, OH, O-C₁-C₄-Alkyl, O-C₁-C₄-Alkyl-Phenyl, NH₂, Phenyl, wobei die Phenyl-Ringe noch mit maximal zwei Resten R²⁴ substituiert sein können, und R²¹ und R²² unabhängig voneinander Wasserstoff oder C₁-C₄-Alkyl bedeuten und R²³ Wasserstoff, C₁-C₄-Alkyl oder Phenyl bedeuten, und R²⁴ OH, C₁-C₆-Alkyl, O-C₁-C₄-Alkyl, Chlor, Brom, Jod, Fluor, CF₃, Nitro, NH₂, und
- x: 0, 1 und 2 sein kann und
- R³: ist B,
- R⁴: Wasserstoff, Chlor, Fluor, Brom, Jod, verzweigtes und unverzweigtes C₁-C₆-Alkyl, OH, Nitro, CF₃, CN, NR⁴¹R⁴², NH-CO-R⁴³, O-C₁-C₄-Alkyl, wobei
R⁴¹ und R⁴² unabhängig voneinander Wasserstoff oder C₁-C₄-Alkyl bedeuten und
R⁴³ Wasserstoff, C₁-C₄-Alkyl, C₁-C₄-Alkyl-Phenyl oder Phenyl bedeuten, und
- B: 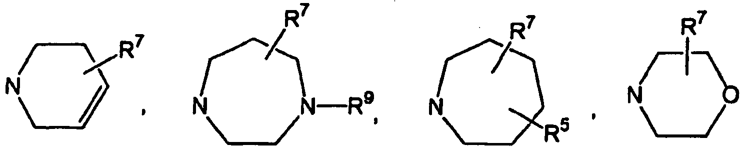 sein kann und
- R⁵: Wasserstoff, C₁-C₆-Alkyl, NR⁷R⁹ und 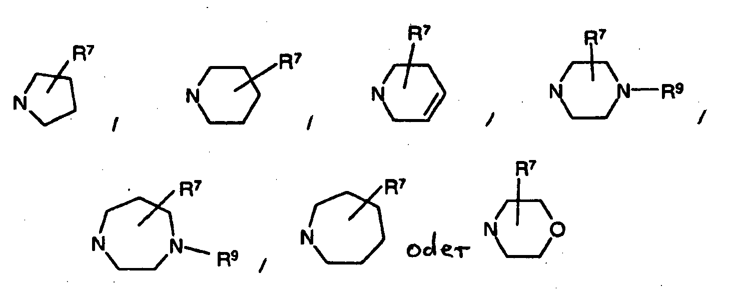 bedeuten kann und
- R⁷: Wasserstoff, C₁-C₆-Alkyl, C₁-C₄-Alkyl-Phenyl, Phenyl, wobei der Ring noch mit bis zu zwei Resten R⁷¹ substituiert sein können, und
- R⁷¹: OH, C₁-C₆-Alkyl, O-C₁-C₄-Alkyl, Chlor, Brom, Jod, Fluor, CF₃, Nitro, NH₂, und
- R⁹: Wasserstoff, COCH₃, CO-O-C₁-C₄-Alkyl, COCF₃, verzweigtes und unverzweigtes C₁-C₆-Alkyl, wobei ein oder zwei Wasserstoffe des C₁-C₆-Alkylrests durch jeweils einen der folgenden Reste substituiert sein kann: OH, O-C₁-C₄-Alkyl und Phenyl und der Phenyl-Ring noch einen oder zwei der folgenden Reste tragen kann: Jod, Chlor, Brom, Fluor, verzweigtes und unverzweigtes C₁-C₆-Alkyl, Nitro, Amino, C₁-C₄-Alkylamino, C₁-C₄-Dialkylamino, OH, O-C₁-C₄-Alkyl, CN, CF₃, SO₂-C₁-C₄-Alkyl, bedeuten kann, und
sowie ihre tautomeren Formen, möglichen enantiomeren und diastereomeren Formen, und pharmakologisch verträglichen Salze.

Besonders bevorzugte Positionen für den Rest R² in der allgemeinen Formel I oder II sind die 3-Position und die 4-Position zum Benzimidazolring. Für den Rest R³ ist ebenfalls die 3-Position oder 4-Position zum Benzimidazolring bevorzugt.

Ganz besonders bevorzugt sind Verbindungen,
wobei R² in 3-Position und R³ in 4-Position oder
R² in 4-Position und R³ in 3-Position zum Benzimidazolring steht.

Die besonders bevorzugte Bedeutung von R¹ ist Wasserstoff.

Die besonders bevorzugte Bedeutung von R² ist Wasserstoff, verzweigtes oder unverzweigtes C₁-C₆-Alkyl, Nitro, CN, NH₂, O-C₁-C₄-Alkyl.
- R⁵: bedeutet bevorzugt einen 6-gliedrigen Ring, insbesondere Piperazin.

Die besonders bevorzugte Bedeutung von R⁴ ist Wasserstoff.

Ganz besonders bevorzugt sind Verbindungen, wobei R¹ und R⁴ Wasserstoff bedeuten.

Ganz besonders bevorzugt sind die jeweiligen Kombinationen der obigen bevorzugten Bedeutungen.

Die Verbindungen der Formel I können als Racemate, als enantiomerenreine Verbindungen oder als Diastereomere eingesetzt werden. Werden enantiomerereine Verbindungen gewünscht, kann man diese beispielsweise dadurch erhalten, daß man mit einer geeigneten optisch aktiven Base oder Säure eine klassische Racematspaltung mit den Verbindungen der Formel I oder ihren Zwischenprodukten durchführt.

Gegenstand der Erfindung sind auch zu Verbindungen der Formel I mesomere oder tautomere Verbindungen.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd. 10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxid und Tris.

Die Herstellung der erfindungsgemäßen 2-Phenylbenzimidazole der Formel I oder II kann auf verschiedenen Wegen erfolgen, die in folgenden Syntheseschemas skizziert wurden.

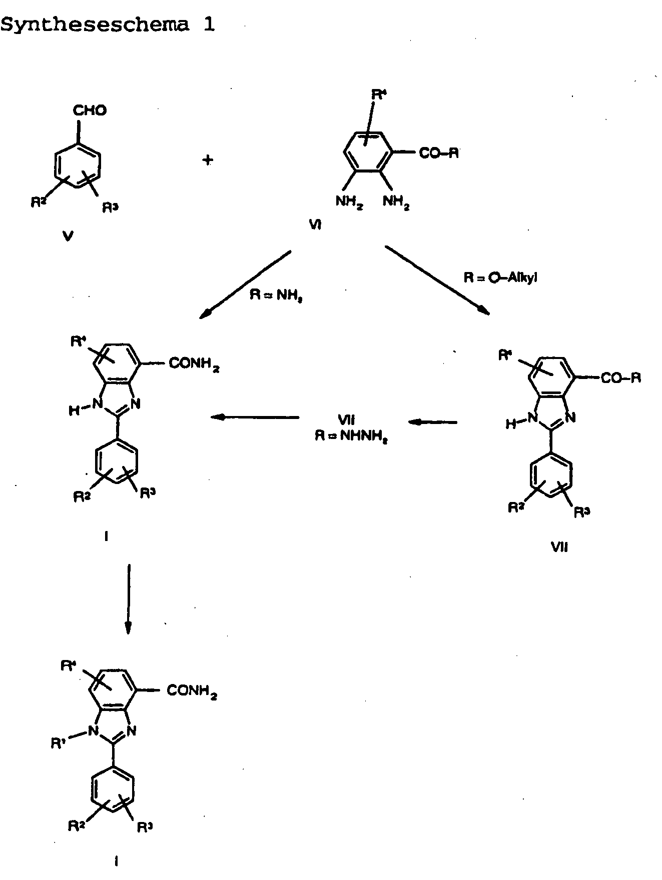

Durch Kondensation von Benzaldehyden V mit Phenylendiaminen VI erhält man das Benzimidazol VII, wobei man bevorzugt in polaren Lösungsmitten wie Ethanol oder Dimethylformamid mit Zusatz von Säuren wie Essigsäure bei erhöhter Temperatur arbeitet, in der Regel 80 bis 120°C. Günstig für die Reaktion ist der Zusatz von schwachen Oxidationsmittel wie Kupfer-II-Salzen, die als wäßrige Lösung zugesetzt werden.

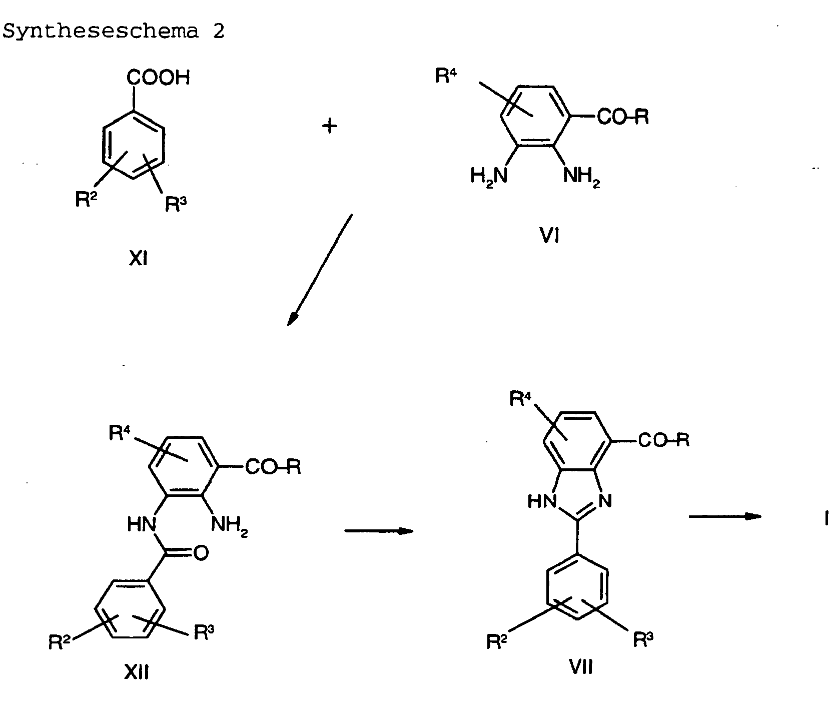

Wenn in dem Phenylendiamin VI R = NH₂ ist, entstehen bei der Kondensation direkt erfindungsgemäße Verbindungen I. Ansonsten kann man, falls R = O-Alkyl ist, diesen Ester mit Ammoniak, bei gegebenenfalls erhöhter Temperatur und erhöhtem Druck, zum Amid I umsetzen. Alternativ kann man den Ester XII mit Hydrazin in polaren Lösungsmitteln, wie die Alkohole Butanol und Ethanol oder auch Dimethylformamid, bei erhöhten Temperaturen, vorzugsweise 80 bis 130°C, umsetzen, wobei ein Hydrazid XII (R = NHNH₂) anfällt, das danach noch unter reduktiven Bedingungen, wie mit Raney-Nickel in Alkoholen unter Rückfluß, zum Amid I reduziert werden kann.

Eine Einführung des Restes R1 am Benzimidazol-Rest in I (R1 =H) gelingt unter üblichen Alkylierungsbedingungen, wie sie zum Beispiel in J.Het.Chem. 1995, 32, 707f und in Tetrahedron 1994, 50, 5535), wobei allerdings der Reaktionspartner R1-L (L = Abgangsgruppe wie Cl, Br. Und J) eingesetzt werden muß.

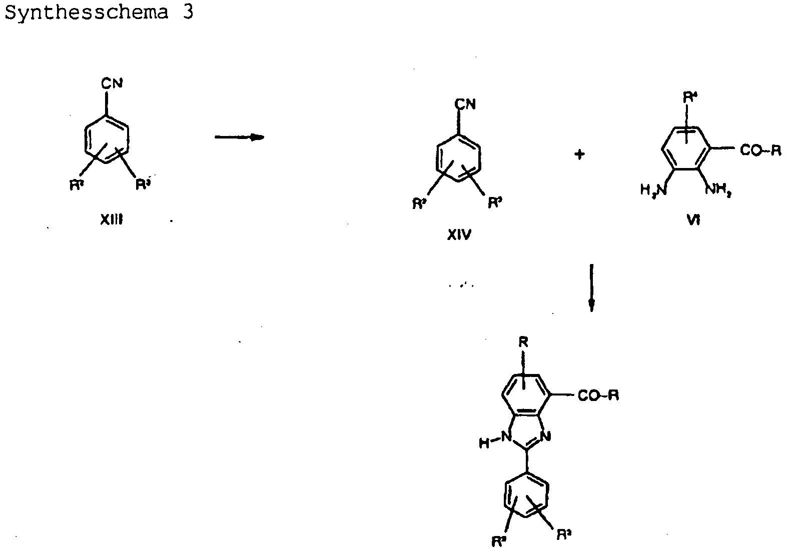

Alternativ zu den im Schema 1 gezeigten Benzaldehyden V kann man auch Benzoesäuren wie XI (siehe Schema 2) oder Benzonitrile wie XIII(siehe Schema 3) anstelle des Benzaldehyds einsetzen. Die Herstellung dieser Derivate erfolgt analog zur Herstellung der substituierten Benzaldehyde V. Ausgehend von XI erfolgt die Kondensation zu VII in zwei Stufen. Zuerst wird die Benzoesäure XI mit dem Anilin VI in einer peptidartigen Kupplung zum Amid XII umgesetzt. Dabei arbeitet man nach üblichen Bedingungen, die zum Beispiel im Houben-Weyl, Methoden der Organischen Chemie, 4. Aufl., E5, Kap.V bzw. C.R. Larock, Comprehensive Organic Transformations, VCH Publisher, 1989, Seite 972f. aufgelistet sind. Der Ringschluß erfolgt zum Benzimidazol erfolgt danach bei erhöhter Temperatur, zum Beispiel 60 bis 180°C, mit oder ohne Lösungsmitteln wie Dimethylformamid, unter Zusatz von Säuren wie Essigsäure oder direkt in Essigsäure selbst.

Die Reaktion des Phenylendiamins VI mit einem Benzonitril XIII erfolgt ebenfalls unter üblichen Bedingungen. Dabei kann man in Lösungsmitteln wie Dimethylformamid unter Zusatz von Säuren bei erhöhter Temperatur, wie 60 bis 200°C, arbeiten. Allerdings kann man auch die üblichen Methoden zur Herstellung von Amidinen aus Benzonitrilen anwenden, wie sie in in Houben-Weyl, Methoden der organischen Chemie, E5, S. 1304 f., J. Amer.Chem.Soc. 1957, 427 und J.Org.Chem. 1987,1017 beschrieben sind.

Die vorliegende Erfindung betrifft außerdem 2,3-Diaminobenzamide der Formel XX, XXI und deren Synthese und Verwendung als Zwischenstufen.

Diaminobenzamide, die am Amid-Rest eine substituierte Alkylkette tragen, werden in WO 9631462 zur Behandlung neurodegenerativer Krankheiten beschrieben. Diaminobenzamide, die am Amid-Rest einen substituierten Aryl-Rest tragen, werden in JP 09059236 zur Behandlung von Entzündungen und Allergien beschrieben. Die Effekte von Benzohydroxamsäuren auf die DNA-Synthese wurden in Bull. Soc. Chim. Belg. 1997, 106, 767 untersucht.

Amino-dibenzodiazepinone wurden in P. V. Khadikar et al., J. Heterocycl. Chem. 1998, 35, 675 hergestellt. Die Synthese von 2-Phenyl-benzimidazyl-4-amiden ist in J. Chem. Soc. Perkin Trans 1, 1979, 2302-2307 beschrieben worden. Analoge Verbindungen, die am Amid-Rest noch eine substituierte Alkyl-Kette tragen, und die cytotoxische Wirkung haben sollen, sind in J. Med. Chem. 1990, 33, 814-819 aufgeführt. In WO 97/04771 sind Benzimidazol-4-amide aufgeführt, die das Enzym PARP hemmen. Insbesondere sind Derivate als wirksam beschrieben, die einen Phenylring in 2-Stellung tragen, wobei der Phenyl-Ring noch mit einfachen Substituenten wie Nitro, Methoxy und CF₃ substituiert sein kann.

Zur Veranschaulichung der Synthesestrategie in WO 97/04771 ist in Schema 4 beispielhaft die Synthese von 2-Phenylbenzimidazol-4-carboxamid (NU 1070) dargestellt.

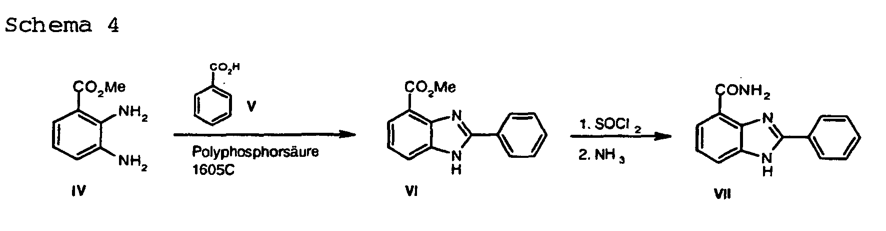

Die Umsetzung von Diaminobenzoesäuremethylester IV mit Benzoesäure V in Polyphosphorsäure ergibt den Benzimidazol-4-carbonsäureester VI in 20 % Ausbeute. Abschließend wird Ester VI mittels Säurechloridbildung in das Amid VII überführt. Für diesen Schritt geben die Autoren eine Ausbeute von 62 % an. Für die Synthesesequenz ergibt sich eine Gesamtausbeute von 12 %. Die Gesamtausbeuten für die Synthesen aller anderen in WO 97/04771 genannten Beispiele liegen in einem Rahmen von 5 bis 19 %. Ein großer Nachteil dieser Synthesestrategie ist die Tatsache, daß jede zu VI analoge Verbindung noch in das Amid überführt werden muß, das erst den wirksamen PARP-Inhibitor darstellt.

Zum Vergleich der Gesamtausbeuten der neuen Synthesestrategie mit denen in WO 97/04771 ist die Synthese von 2-Phenylbenzimidazol-4-carboxamid in Schema 11 dargestellt. Die Umsetzung von Ester XIV zum Amid XV erfolgt in 70 %. Die Synthese des Benzimidazols VII durch Kondensation von XV mit Benzaldehyd XVI mit anschließender Oxidation verläuft mit 85 % Ausbeute. Die daraus resultierende Gesamtausbeute von 60 % übertrifft die entsprechende Gesamtausbeute von 12 % in WO 97/04771.

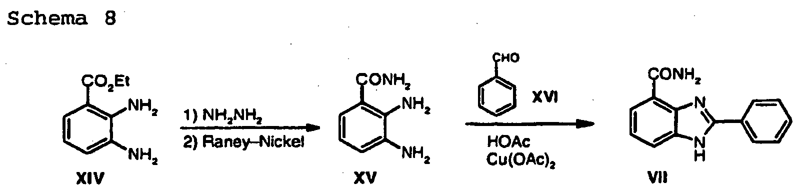

Die in der vorliegenden Erfindung enthaltenen substituierten 2-Phenylbenzimidazole I oder II stellen Inhibitoren des Enzyms Poly-(ADP-ribose)polymerase oder PARP (EC 2.4.2.30) dar.
Die inhibitorische Wirkung der substituierten 2-Phenylbenzimidazole I oder II wurde mit einem in der Literatur bereits bekannten Enzymtest ermittelt, wobei als Wirkmaßstab ein Ki-Wert ermittelt wurde. Die 2-Phenylbenzimidazole I wurden in dieser Weise auf Hemmwirkung des Enzyms Poly(ADP-ribose)polymerase oder PARP (EC 2.4.2.30) gemessen.

### Bestimmung des Wasserlöslichkeit

Eine zu messende Verbindung wird direkt in einem festgelegten Volumen Wasser gelöst und die entstandene Lösung mit einer Natriumacetat-Lösung auf pH 5 bis 6 eingestellt, so daß die zu prüfende Konzentration des Wirkstoffs erreicht wird. Falls die Meßsubstanz nicht als wasserlösliches Salz vorliegt, wurde diese in möglichst wenig Dimethylsulfoxid gelöst und anschließend mit Wasser verdünnt (Endkonzentration an Dimethylsulfoxid 5 1 %), wonach auch hier der pH-Wert noch eingestellt wurde. Der potente PARP-Inhibitor NU 1076 (WO 97/04771) zeigte hier eine Löslichkeit < 0,01 %, wogegen das erfindungsgemäße Beispiel 2 eine Löslichkeit > 0,5 % aufweist.

Die substituierten 2-Phenylbenzimidazole der allgemeinen Formeln I stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten Enzymaktivität dieser Enzyme verbunden sind, dienen.

Die Verbindungen der Formeln I können zur Herstellung von Arzneimitteln zur Behandlung von Schädigungen nach Ischämien und zur Prophylaxe bei erwarteten Ischämien verschiedener Organe eingesetzt werden.

Die Verbindungen der Formel I können eingesetzt werden zur Herstellung von Arzneimitteln zur Behandlung von Krankheiten, bei denen pathologisch erhöhte Aktivitäten von PARP auftreten; bevorzugt zur Behandlung von neuro-degenerativen Krankheiten und neuronalen Schädigungen oder zur Behandlung von solchen neuro-degenerativen Krankheiten und neuronalen Schädigungen, die durch Ischämie, Trauma oder Massenblutungen ausgelost werden.

Außerdem können die Verbindungen der Formel I eingesetzt werden zur Herstellung von Arzneimitteln zur Behandlung des Schlaganfalls und des Schädel-Himtraumas, zur Behandlung der Alzheimerschen Krankheit, der Parkinsonsche Krankheit und der Huntington-Krankheit, zur Behandlung oder Prophylaxe von Schädigungen durch Ischämien, zur Behandlung von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen, zur Behandlung von Schädigungen der Nieren nach renalen Ischämien, Schädigungen, die durch medikamentöse Therapie verursacht werden, wie zum Beispiel während der Cyclosporin-Therapie, und zur Behandlung während und nach Nierentransplantationen, zur Behandlung von Schädigungen des Herzens nach cardialen Ischämien, zur Behandlung von Mikroinfarkten wie zum Beispiel während und nach Herzklappenersatz, Aneurysmenresektionen und Herztransplantationen, zur Behandlung bei einer Revasculariation kritischer verengter Koronararterien wie zum Beispiel bei PTCA und Bypass-Operationen oder kritisch verengter peripherer Arterien, insbesondere Beinarterien, zur Behandlung des akuten Myocardinfarktes und von Schädigungen während und nach dessen medikamentöser oder mechanischer Lyse, zur Behandlung von Tumoren und deren Metastasierung, zur Behandlung von Sepsis des Multiorganversagens wie zum Beispiel während des septischen Schocks und des "acute respiratory distress-syndroms", zur Behandlung von immunologischen Krankheiten wie Entzündungen und rheumatische Erkrankungen, wie zum Beispiel rheumatoide Arthritis und zur Behandlung von Diabetes mellitus.

Die vorliegenden 2-Phenylbenzimidazole der allgemeinen Formel I können danach zur Behandlung und Prophylaxe von neurodegenerativen Krankheiten, die nach Ischämie, Trauma (Schädel-Hirntrauma), Massenblutungen, Subarachnoidal-Blutungen und Stroke auftreten, und von neurodegenerativen Krankheiten, wie multipler Infarkt-Dementia, Alzheimer Krankheit, Huntington Krankheit und von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, des akuten Nierenversagens oder von Schädigungen, die während und nach einer Nierentransplantation auftreten, dienen. Weiterhin können die Verbindungen der allgemeinen Formel I zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator), und von Mikroinfarkten während und nach Herzklappenersatz, Aneurysmenresektionen und Herztransplantationen dienen. Zudem können die 2-Phenylbenzimidazole I bei der Chemotherapie von Tumoren und deren Metastasierung nützlich sein und zur Behandlung von Entzündungen und rheumatischen Erkrankungen, wie z.B. rheumatischer Arthritis, dienen.

Neue PARP-Inhibitoren können in relevanten pharmakologischen Modellen auf ihre therapeutische Wirksamkeit überprüft werden. Beispiele für einige geeignete Modelle sind dazu in Tabelle 1 aufgeführt.

**Tabelle 1**

| Krankheit | Modell | Literatur |
|---|---|---|
| Neurodegenerative Erkrankungen (Schlaganfall, Parkinson etc.) | NMDA-Exzitotoxizität in der Maus oder Ratte | |
| Schlaganfall | Permanente MCAO ("middle cerebral artherial occlusion") | Tokime T. et al., J. Cereb Blood Flow Ketab, 18(9):991-7, 1998 Guegan C. Brain Research. Molecular Brain Research 55(1) 133-40, 1998 |
| | Transiente, fokale MCAO in ratte oder Maus | Eliasson MJL et al., Nat Med 1997, 3:1089-1095. Endres M. et al., J. Cereb Blood Flow Metab 1997, 17:1143-1151. Takahashi K. et al., J. Cereb Blood Flow Metab 1997, 17:1137-1142. |
| Parkinsonsche Krankheit | MPTP (1-Methyl-4-phenyl-1,2,3,6-tetrahydro-pyridin) Toxizität in der Maus/Ratte | Cosi C. et al., Brain Res. , 1998 809(1):58-67. Cosi C. et al. , Brain Res., 1996 729(2):264-9. |
| Herzinfarkt | Koronargefäß-Okklusion an Ratte, Schwein oder Kaninchen | Richard V. et al., Br. J. Pharmacol 1994, 113, 869-876. |
| | | Thiemermann C. et al., Proc Natl Acad Sci USA. 1997, 94(2):679-83. |
| | | Zingarelli B. et al., Cardiovasc Res. 1997, 36(2):205-15. |
| | Langendorfherzmodell an Ratte oder Kaninchen | Beschreibung siehe unten |
| Septischer Schock | Endotoxin Schock in der Ratte | Szabo C. et al., J. Clin Invest, 1997, 100(3):723-35. |
| | Zymosan oder Carrageenan induziertes multiples Organversagen in Ratte oder Maus | Szabo C. et al. J. Exp Med. 1997, 186(7):1041-9. Cuzzocrea S. et al. Eur J. Pharmacol. 1998, 342(1):67-76. |
| Rheumatoide Arthritis | Adjuvant oder Collagen induzierte Arthritis in Ratte oder Maus | Szabo C. et al., Proc Natl Acad Sci USA. 1998, 95(7):3867-72. |
| Diabetes | Streptozotocin und Alloxan induiziert bzw. Obesity assoziert | Uchigata Y. et al., Diabetes 1983, 32: 316-318. Masiello P. et al., Dia-betologia 1985, 28: 683-686. Shimabukuro M. et al., J. Clin Invest 1997, 100: 290-295. |
| Krebs | | Schlicker et al. 1999 75:1, 91-100 |

Die erfindungsgemäßen Arzneimittelzubereitungen enthalten neben den üblichen Arzneimittel-Hilfsstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-%, enthalten.

Bei der inneren Anwendung werden die Präparationen in Einzeldosen verabreicht. In einer Einzeldosis werden pro kg Körpergewicht 0,1 bis 100 mg gegeben. Die Zubereitungen können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intra-peritoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiel 1

### 2(4(2-(N,N-Diethylamino)eth-1-yloxy)phenyl)-benzimidazol-4-carbonsäureamid

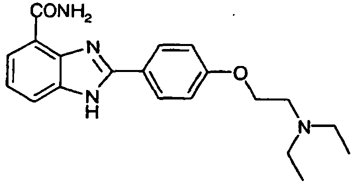

### a) 4(2-(N,N-Diethylaminoeth-1-yloxy)-benzaldehyd

15 g (122 mMol) 4-Hydroxybenzaldehyd, 16,7 g (122 mMol) N(2-Chlorethyl)-N,N-diethylamin und 33,9 g (246 mMol) Kaliumkarbonat wurden zusammnen mit einer Spatelspitze 18-Krone-6 in 300 ml Ethylmethylketon für 6 Stunden unter Rückfluß gekocht. Anschließend wurde filtriert und das Filtrat im Vakuum eingeengt. Der Rückstand wurde zwischen Ether und 2 M Natronlauge verteilt, die Ether-Phase abgetrennt, getrocknet und im Vakuum eingeengt. Man erhielt 24,8 g des Zwischenproduktes.

### b) 2(4(2-(N,N-Diethylamino)eth-1-yloxy)phenyl)-benzimidazol-4-carbonsäureethylester

2 g (11 mMol) 2,3-Diaminobenzoesäureethylester und 1,4 ml konzentrierte Essigsäure wurden in 25 ml Methanol gelöst. Anschließend wurden 3,2 g (14,4 mMol) der Zwischenverbindung 1a, gelöst in 50 ml Methanol, innerhalb von 30 Minuten zugetropft. Danach wurden 2,9 g (14,4 mMol) Kupfer-II-acetat, gelöst in 37,5 ml warmen Wasser, zügig zugetropft und anschließend alles für 20 Minuten unter Rückfluß gekocht. Man kühlte die Reaktionslösung auf 50oC und gab 4,5 ml 32%iger Salzsäure zu. Danach tropfte man noch vorsichtig eine Lösung aus 4,3 g Natriumsulfid-Hydrat in 25 ml Wasser zu und rührte alles noch für 15 Minuten. Die Reaktionslösung wurde auf Eiswasser gegossen und der anfallende Niederschag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 4,4 g des Zwischenproduktes.

### c) 2-(4(2-(N,N-Diethylamino)eth-1-yloxy)phenyl)-benzimidazol-4-carbonsäurehydrazid

Zu 4,1 g (10,7 mMol) der Zwischenverbindung 1b in 30 ml Ethanol wurden 2,7 g (54 mMol) hydrazinhydrat gegeben und alles für 10 Stunden unter Rückfluß gekocht. Anschließend wurde das organische Lösungsmittel im Vakuum entfernt und der Rückstand zwischen Wasser und Essigester verteilt. Die Essigester-Phase wurde abgetrennt, getrocknet und im Vakuum eingeengt. Der so erhaltene Rückstand wurde noch mit Ether behandelt und erneut abgesaugt, wonach man 1.7g der Zwischenverbindung.

### d) 2(4(2-(N,N-Diethylamino)eth-1-yloxy)phenyl)-benzimidazol-4-carbonsäureamid

Zu 1,6 g (4,5 mMol) der Zwischenverbindung 1c in 45 ml Dimethylformamid/Wasser (2/1) wurden ca. 1,6 g Raney-Nickel gegeben und alles für 6 Stunden auf 100°C erwärmt. Anschließend wurde das Reaktionsgemisch filtriert und das Filtrat mit viel Wasser verdünnt, wobei das Produkt ausfiel. Man erhielt 1,2 g des Produktes.

¹H-NMR (D₆-DMSO). δ = 0,95(6H), 2,6(4H), 2,8(2H), 4,1(2H), 7,1(2H), 7,3(1H), 7,7(1H + NH), 7,85(1H), 8,2(2H) und 9,4 (NH)ppm.

### Beispiel 2

### 2 (4 (2-(N,N-Diethylamino)eth-1-yloxy)phenyl)-benzimidazol-4-carbonsäureamid x 2 Hydrochlorid

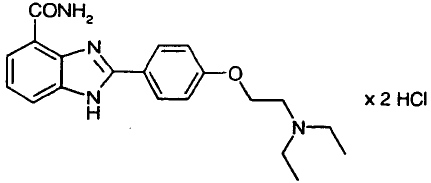

0,2g des Produktes aus Beispiel 1 wurden in einem Gemisch aus Essigester und wenig Tetrahydrofuran gelöst und mit etherischer Chlorwasserstoff-Lösung versetzt, wobei sich ein Niederschlag bildet. Dieser Niederschlag wurde abgesaugt, mit Aceton aufgeschlämmt und erneut abgesaugt, wonach man ca. 200 mg des Produktes erhielt.

¹H-NMR (D₆-DMSO). δ = 1,2(6H), 3,2(4H), 3,3(2H), 4,5(2H), 7,25(1H), 7,4(1H), 7,8-7,9(2H), 8,3(2H), 9,0(NH) und 10, 5 (NH) ppm.

### Beispiel 3

### 2(3(2-(N,N-Diethylamino)eth-1-yloxy)phenyl)-benzimidazol-4-carbonsäureamid

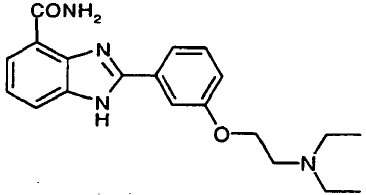

### a) 3(2-(N,N-Diethylamino eth-1-yloxy)-benzaldehyd

6,1 g (50 mMol) 3-Hydroxybenzaldehyd wurden in 100 ml Ethanol gelöst und 3,5 g (50 mMol) Natriumethanolat zugegeben. Man rührte alles für 15 Minuten. Danach wurden 7,5 g (55 mMol) N(2-Chlorethyl)-N,N-diethylamin zugefügt und alles für 12 Stunden unter Rückfluß gekocht. Anschließend wurde das Reaktionsgemisch im Vakuum eingeengt. Der Rückstand wurde daanch zwischen Ether und 1M Natronlauge verteilt, die Ether-Phase abgetrennt, getrocknet und im Vakuum eingeengt. Man erhielt 7.6g des Zwischenproduktes.

### b) 2 (3 (2-(N,N-Diethylamino)eth-1-yloxy)phenyl)-benzimidazol-4-carbonsäureethylester

1 g (5,5 mMol) 2,3-Diaminobenzoesäureethylester und 0,68 ml konzentrierte Essigsäure wurden in 20 ml Methanol gelöst. Anschließend wurden 1,6 g (7,2 mMol) der Zwischenverbindung 3a, gelöst in 30 ml Methanol, innerhalb von 30 Minuten zugetropft. Danach wurden 1,1 g (5,5 mMol) Kupfer-II-acetat, gelöst in 19 ml warmen Wasser, zügig zugetropft und anschließend alles für 20 Minuten unter Rückfluß gekocht. Man kühlte die Reaktionslösung auf 50oC und gab 2,25 ml 32%iger Salzsäure zu. Danach tropfte man noch vorsichtig eine Lösung aus 2,13 g Natriumsulfid-Hydrat in 15 ml Wasser zu und rührte alles noch für 15 Minuten. Die Reaktionslösung wurde auf Eiswasser gegossen und der anfallende Niederschag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 2,4 g des Zwischenproduktes.

### c) 2 (3 (2-(N,N-Diethylamino)eth-1-yloxy)phenyl)-benzimidazol-4-carbonsäurehydrazid

Zu 2,3 g (6,0 mMol) der Zwischenverbindung 3b in 30 ml Butanol wurden 1,5 g (30 mMol) hydrazinhydrat gegeben und alles für 10 Stunden auf 120°C erwärmt. Anschließend wurde das Reaktionsgemisch mit viel Wasser verdünnt und mit Essigester extrahiert. Die Essigester-Phase wurde abgetrennt, getrocknet und im Vakuum eingeengt. Man erhielt 1,7 g der Zwischenverbindung.

### d) 2(3(2-(N,N-Diethylamino)eth-1-yloxy)phenyl)-benzimidazol-4-carbonsäureamid

Zu 1 g (2,7 mMol) der Zwischenverbindung 3c in 30 ml Dimethylformamid/Wasser (2/1) wurden ca. 1,5 g Raney-Nickel gegeben und alles für 6 Stunden auf 100°C erwärmt. Anschließend wurde das Reaktionsgemisch filtriert und das Filtrat mit viel Wasser verdünnt, wobei das Produkt ausfiel. Man erhielt 0,74 g des Produktes. ¹H-NMR (D₆-DMSO). δ = 1,0(6H), 2,6(4H), 2,9(2H), 4,15(2H), 7,1(1H), 7,4(1H), 7,5(1H), 7,7-7,9 (5H) und 9,3 (NH) ppm.

### Beispiel 4

### 2(3(2-(N,N-Diethylamino)eth-1-yloxy)phenyl)-benzimidazol-4-carbonsäureamid x 2 Hydrochlorid

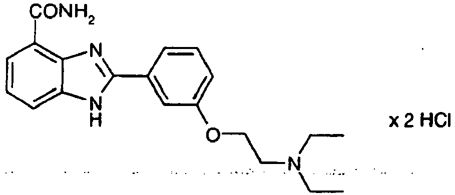

0,2 g des Produktes aus Beispiel 3 wurden in einem Gemisch aus Essigester und Tetrahydrofuran gelöst und mit etherischer Chlorwasserstoff-Lösung versetzt, wobei sich ein Niederschlag bildet. Dieser Niederschlag wurde abgesaugt, mit Aceton aufgeschlämmt und erneut abgesaugt, wonach man ca. 200 mg des Produktes erhielt. ¹H-NMR (D₆-DMSO). δ = 1,3(6H), 3,2(4H), 3,6(2H), 4,6(2H), 7,2-8,1(8H), 9,0(1H), und 10,8(NH) ppm.

Analog dem Beispiel 1 wurden hergestellt:

### Beispiel 5

### 2(3 (2-(N,N-Dimethylamino)eth-1-yloxy)phenyl)-benzimidazol-4-carbonsäureamid

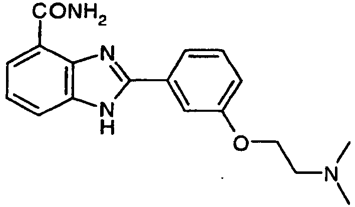

¹H-NMR (D₆-DMSO). δ = 2,2(6H), 2,7(2H), 4,2(2H), 7,0-8,0(9H) und 9,3(1H) ppm.

### Beispiel 6

### 2(3(2-(N,N-Dimethylamino)eth-1-yloxy)-4-methoxy-phenyl)-benzimidazol-4-carbonsäureamid

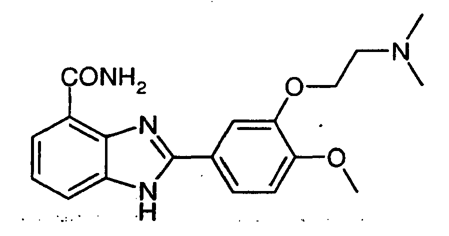

¹H-NMR (D₆-DMSO). δ = 2,25(6H), 2,75(2H), 3,8(3H), 4,1(2H), 7,0-8,1(8H) und 9,4(1H) ppm.

### Beispiel 7

### 2(3(2-(N,N-Dimethylamino)eth-1-yloxy)-4-methoxy-phenyl)-benzimidazol-4-carbonsäureamid x 2 HCl

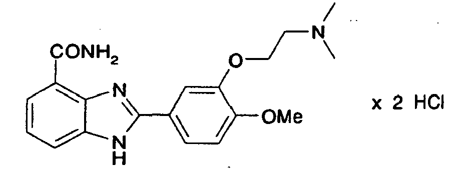

¹H-NMR (D₂O): δ = 3,0(6H), 3,7(2H), 3,8(3H), 4,3(2H), 6,9(1H), 7,3(1H), 7,3-7,5(3H) und 7,7(3H) ppm.

### Beispiel 8

### 2(2(2-(N,N-Dimethylamino)eth-1-yloxy)-phenyl)-benzimidazol-4-carbonsäureamid x 2 HCl

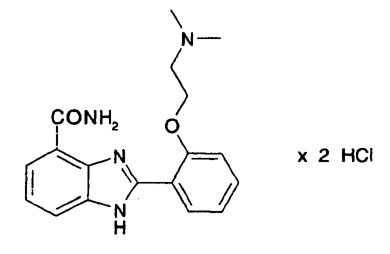

¹H-NMR (D₆-DMSO): δ = 2,9(6H), 3,7(2H), 4,7(2H), 7,2-8,3(8H), 8,9(breit) und ca 11(breit) ppm.

### Beispiel 9

### 2(3(2-(N,N-Dimethylamino)eth-1-yloxy)-phenyl)-benzimidazol-4-carbonsäureamid x 2 Hydrochlorid

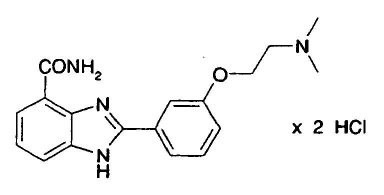

¹H-NMR (D₆-DMSO): δ = 2,9(6H), 3,5(2H), 4,5(2H), 7,2-8,1(8H), 9,0(breit) und ca 10,8(breit) ppm.

### Beispiel 10

### 2 (3 (3 - (tert. -Butoxycarbonylamino) prop-1-yl oxy) -phenyl) -benzimidazol-4-carbonsäureamid

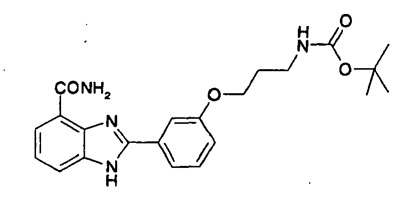

¹H-NMR (D₆-DMSO): δ = 1,3(9H), 1,9(2H), 3,1(2H), 4,1(2H), 6,9-8,0(9H) und ca 9,3(breit) ppm.

### Beispiel 11

### 2(3(3-(tert.-Butoxycarbonylamino)eth-1-yloxy)-phenyl)-benzimidazol-4-carbonsäureamid

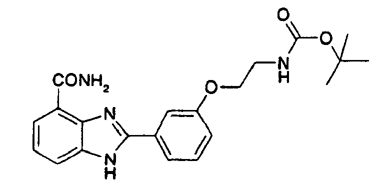

¹H-NMR (D₆-DMSO): δ = 1,3(9H), 3,3(2H), 4,1(2H), 7,0-8,0(9H) und ca 9,3(breit) ppm.

### Beispiel 12

### 2(3(3-(4(3-Chlorphenyl)piperazin-1-yl)prop-1-yloxy)-phenyl)-benzimidazol-4-carbonsäureamid

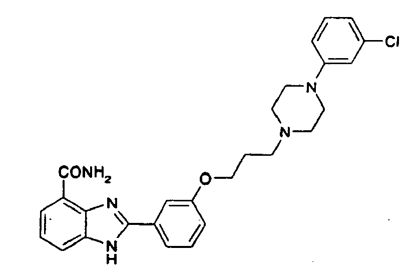

¹H-NMR (D₆-DMSO): δ = 2,3(2H), 3,3-3,5(6H), 3,7(2H), 3,7-4,3(6H), 6,9-8,0(11H), 9,1(breit) und ca 10,9(breit) ppm.

### Beispiel 13

### 2(3(3-(N,N-Diethylamino)prop-1-yloxy)-phenyl)-benzimidazol-4-carbonsäureamid x 2 HCl

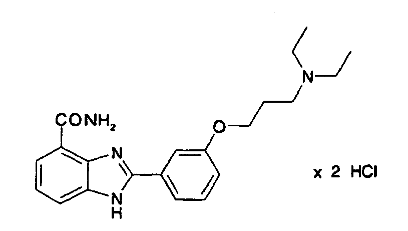

¹H-NMR (D₆-DMSO): δ = 1,2(6H), 2,2(2H), 3,2(4H), 3,8(2H), 4,3(2H), 7,1-8,0(7H), 9,1(breit) und ca 10,5(breit) ppm.

### Beispiel 14

### 2(3(3-Aminoprop-1-yloxy)-phenyl)-benzimidazol-4-carbonsäureamid x 2HCl

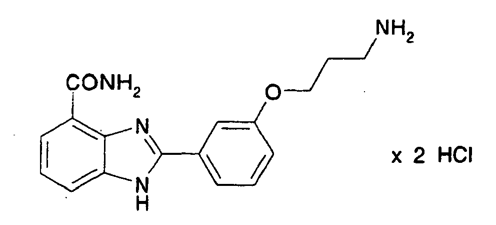

¹H-NMR (D₆-DMSO): δ = 2,1(2H), 3,0(2H), 4,2(2H), 7,2(1H), 7,5(2H), 7,8-8,1(6H), 8,2(breit) und ca 8,9(breit) ppm.

### Beispiel 15

### 2(3(2-Aminoeth-1-yloxy)-phenyl)-benzimidazol-4-carbonsäureamid x 2HCl

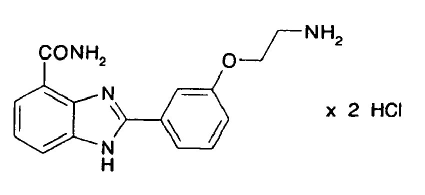

¹H-NMR (D₆-DMSO): δ = 3,2(2H), 4,2(2H), 7,1-8,0(9H), 8,2 (breit) und 9,0(breit) ppm.

Folgende Beispiele können analog der obigen Vorschriften hergestellt werden.

### Beispiel 16

2(4(3-(N,N-Diethylamino)prop-1-yloxy)-phenyl)-benzimidazol-4-carbonsäureamid x 2 HCl
¹H-NMR (D₆-DMSO): δ = 1,3(6H), 2,2(2H), 3,2(6H), 4,2(2H), 7,2(2H), 7,5(1H), 7,8-8,0(3H), 8,35(2H), 8,9(1H) und 10,7(breit) ppm.

### Beispiel 17

1-(3(N,N-Diethylamino)-prop-1-yl)-2(4(3-(N,N-diethylamino)prop-1-yloxy)-phenyl)-benzimidazol-4-carbonsäureamid x 2 HCl
¹H-NMR (D₆-DMSO): δ = 1,1-1,3(12H), 2,2(4H), 2,9-3,3(12H), 4,2(2H), 4,5(2H), 7,2(2H), 7,6(1H), 7,8-8,1(3H), 8,3(1H), 8,4(1h), 8,9(1H)und 11,0(breit) ppm.

### Beispiel 18

2(4(2-(Pyrrolidin-lyl)-eth-1-yloxy)-phenyl)-benzimidazol-4-carbonsäureamid x 2 HCl
¹H-NMR (D₆-DMSO): δ = 1,3(1H), 1,7-2,0(5H), 3,0(2H), 3,5(4H), 4,5(2H), 7,2(2H), 7,3(1H), 7,7-8,0(3H), 8,2(2H), 8,9(breit) und 10,7(breit) ppm.

### Beispiel 19

1-(3(Pyrrolidin-1-yl)-prop-1-yl)-2(4(2-(pyrrolidin-1yl)-eth-1-yloxy)-phenyl)-benzimidazol-4-carbonsäureamid x 2 HCl
¹H-NMR (D₆-DMSO): δ = 1,3(2H), 1,7-1,9(10H), 3,0(4H), 3,3-3,6(8H), 4,5(2H), 4,9(2H), 7,1(2H), 7,5(1H), 7,7-8,0(3H), 8,1(2H), 9,0(breit), 10,8(breit) und 11,2(breit) ppm.

### Beispiel 20

2(4(3(N,N-Benzylmethylamino)-prop-1-yloxy)-phenyl)-benzimidazol-4-carbonsäureamid x 2 HCl

### Beispiel 21

1(3(N,N-Benzylmethylamino)-prop-1-yl)-2(4(3(N,N-benzylmethylamino)-prop-1-yloxy)-phenyl)-benzimidazol-4-carbonsäureamid x 2 HCl
MS: m/e = 575 (M⁺).

### Beispiel 22

2(4(3 (4-Methylpiperazin-1-yl)-prop-1-yloxy)-phenyl)-benzimidazol-4-carbonsäureamid x 3 HCl
MS: m/e = 393 (M⁺).

### Beispiel 23

2 (3 (2 (N,N-Benzylmethylamino)-eth-1-yloxy)-4-nitrophenyl)-benzimidazol-4-carbonsäureamid
¹H-NMR (D₆-DMSO): δ = 1,0(6H), 2,5-2,8(4H), 2,9(2H), 4,3(2H), 7,3(1H), 7,8-8,2(6H) und 9,1(1H) ppm.

### Beispiel 24

### 2(4(3-Trifluoracetamidomethyl-pyrrol-1-yl)phenyl)-benzimidazol-4-carbonsäureamid

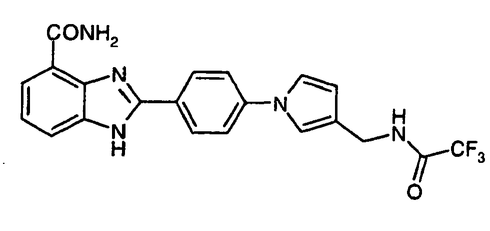

a) 2 (4-Nitrophenyl)-benzimidazol-4-carbonsäureethylester
   1,5 g (8,3 mMol) 2,3-Diaminobenzoesäureethylester und 1,1 ml konzentrierte Essigsäure wurden in 50 ml Methanol gelöst. Anschließend wurden 1,6 g (10,8 mMol) 4-Nitrobenzaldehyd, gelöst in 150 ml Methanol, innerhalb von 30 Minuten zugetropft. Danach wurden 2,2 g (10,8 mMol) Kupfer-II-acetat, gelöst in 100 ml warmen Wasser, zügig zugetropft und anschließend alles für 20 Minuten unter Rückfluß gekocht. Man kühlte die Reaktionslösung auf 50oC und gab 3 ml 32%iger Salzsäure zu. Danach tropfte man noch vorsichtig eine Lösung aus 3,1 g Natriumsulfid-Hydrat in 50 ml Wasser zu und rührte alles noch für 15 Minuten. Die Reaktionslösung wurde auf Eiswasser gegossen und der entstandene Niederschlag abgesaugt. Das Filtrat wurde mit wäßriger Natriumhydrogenkarbonat-Lösung alkalisch gestellt und mehrmals mit Essigester extrahiert.
   Die Essigester-Phase wurde abgetrennt, getrocknet und im Vakuum eingeengt. Man erhielt 2,2 g des Zwischenproduktes.
b) 2(4(4-Nitro-phenyl)-benzimidazol-4-carbonsäurehydrazid
   Zu 2,1 g (6,7 mMol) der Zwischenverbindung 24a in 25 ml Ethanol wurden 1,7 ml (34 mMol) Hydrazinhydrat gegeben und alles für 4 Stunden unter Rückfluß gekocht. Anschließend wurde das organische Lösungsmittel im Vakuum entfernt und der Rückstand zwischen Wasser und Essigester verteilt. Die Essigester-Phase wurde abgetrennt, getrocknet und im Vakuum eingeengt. Der so erhaltene Rückstand wurde noch mit Ether behandelt und erneut abgesaugt, wonach man 1,7 g der Zwischenverbindung erhielt.
c) 2(4(4-Amino-phenyl)-benzimidazol-4-carbonsäureamid
   Zu 1,7 g (5,7 mMol) der Zwischenverbindung 24b in 120 ml Ethanol/Essigsäure (5/1) wurden ca. 1 g Palladium-Kohle (10%ig) und alles mit Wasserstoff hydriert. Anschließend wurde das Reaktionsgemisch filtriert und im Vakuum eingeengt. Der Rückstand wurde in 70 ml eines Gemisches aus Dimethylformamid und Wasser (7/3) gelöst. Anschließend wurden 2 g Raney-Nickel zugegeben und alles für 4 h auf 100°C erwärmt. Danach wurde das Reaktionsgemisch filtriert und das Filtrat im Vakuum eingeengt. Der so erhaltene Rückstand wurde in Ether dispergiert und abgesaugt, wobei man 1,5 g des Produktes erhielt.
d) 2 (4(3-Trifluoracetamidomethyl-pyrrol-1-yl)phenyl)-benzimidazol-4-carbonsäureamid
   1,4 g (5,6 mMol) der Zwischenverbindung 24c und 1,8 g (6,9 mMol) 2,5-Dimethoxy-3-(trifluoracet-amidomethyl)tetrahydrofuran wurden in 50 ml konzentrierter Essigsäure gegeben und für 10 Minuten unter Rückfluß erwärmt. Anschließend wurde alles im Vakuum eingeengt und der anfallende Rückstand chromatographisch an Kieselgel mit dem Fließmittel Essigester gereinigt. Man erhielt 1,9 g des Produktes.
   ¹H-NMR (D₆-DMSO). δ = 4,3(2H), 6,3(1H), 7,35(1H), 7,5(1H), 7,7-7,9(5H), 8,3(2H), 9,4(1H) und 9,9(1H) ppm.

### Beispiel 25

### 2(4(3-Aminomethyl-pyrrol-1-yl)phenyl)-benzimidazol-4-carbonsäureamid

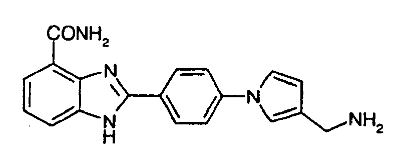

1,7 g (4 mMol) des Beispiels 24 wurden in 70 ml Tetrahydrofuran gelöst und mit einer Lösung aus 0,38 g (15,9 mMol) Lithiumhydroxid in 25 ml Wasser versetzt. Alles wurde für 2 Stunden bei Raumtemperatur gerührt. Anschließend wurde das Reaktionsgemisch mit verdünnter Salzsäure neutralisiert und das organische Lösungsmittel im Vakuum entfernt. Der anfallende Niederschlag wurde abgesaugt und getrocknet. Man erhielt 0,87 g des Produktes.
¹H-NMR (D₆-DMSO). δ = 4,4(2H), 7,0(NH) und 7,8-8,4(11H) ppm.

### Beispiel 26

### 2(4(3-Aminomethyl-pyrrol-1-yl)phenyl)-benzimidazol-4-carbonsäureamid x 2 Methan-sulfonsäure

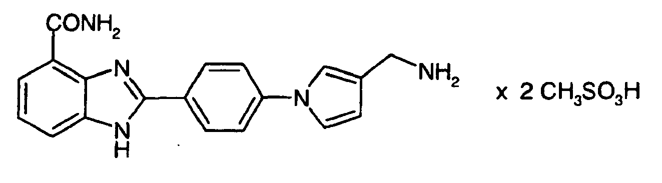

0,1 g des Produktes aus Beispiel 25 wurden in 2 ml Tetrahydrofuran gelöst und mit 20,5 µL Methansulfonsäure, verdünnt mit 5 ml Wasser, versetzt. Man verdünnte anschließend noch mit Wasser und lyophilisierte danach die so erhaltene Lösung, wobei man 117 mg des Produktes erhielt.
¹H-NMR (D₆-DMSO). δ = 2,45(6H), 4,0(2H), 6,4(1H), 7,2-8,4(11H) und 9,1(NH) ppm.

### Beispiel 27

### 2(4(1-Imidazolyl)phenyl)-benzimidazol-4-carbonsäureamid

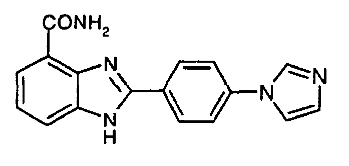

a) 2 (4(1-Imidazolyl)phenyl)-benzimidazol-4-carbonsäureethylester
   1 g (5,5 mMol) 2,3-Diaminobenzoesäureethylester und 0,7 ml konzentrierte Essigsäure wurden in 13 ml Methanol gelöst. Anschließend wurden 1,24 g (7,2 mMol) 4-Imidazol-1-ylbenzaldehyd, gelöst in 25 ml Methanol, innerhalb von 30 Minuten zugetropft. Danach wurden 1,4 g (7,2 mMol) Kupfer-II-acetat, gelöst in 19 ml warmen Wasser, zügig zugetropft und anschließend alles für 20 Minuten unter Rückfluß gekocht. Man kühlte die Reaktionslösung auf 50oC und gab 2,25 ml 32%iger Salzsäure zu. Danach tropfte man noch vorsichtig eine Lösung aus 2,13 g Natriumsulfid-Hydrat in 15 ml Wasser zu und rührte alles noch für 15 Minuten. Die Reaktionslösung wurde auf Eiswasser gegossen und der anfallende Niederschag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 1,7 g des Zwischenproduktes.
b) 2(4(1-Imidazolyl)phenyl)-benzimidazol-4-carbonsäurehydrazid
   Zu 1,6 g (5,0 mMol) der Zwischenverbindung 27a in 30 ml Butanol wurden 5 ml Hydrazinhydrat gegeben und alles für 8 Stunden unter Rückfluß gekocht. Anschließend wurde das Reaktionsgemisch im Vakuum eingeengt und der Rückstand zwischen Wasser und Essigester verteilt. Die Essigester-Phase wurde abgetrennt, getrocknet und im Vakuum eingeengt. Man erhielt 0,55 g der Zwischenverbindung.
c) 2(4(1-Imidazolyl)phenyl)-benzimidazol-4-carbonsäureamid
   Zu 0,53 g (1,7 mMol) der Zwischenverbindung 27b in 35 ml Dimethylformamid/Wasser (2/1) wurden ca. 1,5 g Raney-Nickel gegeben und alles für 8 Stunden auf 100°C erwärmt. Anschließend wurde das Reaktionsgemisch filtriert und das Filtrat mit viel Wasser verdünnt, wobei das Produkt ausfiel. Man erhielt 0,19 g des Produktes.
   ¹H-NMR (D₆-DMSO). δ = 7,2(1H), 7,4(1H), 7,7-8,0(6H) 8,4(3H) und 9,4(1H) ppm.

### Beispiel 28

### 2(4(1-Imidazolyl)phenyl)-benzimidazol-4-carbonsäureamid x 2Methansulfonsäure

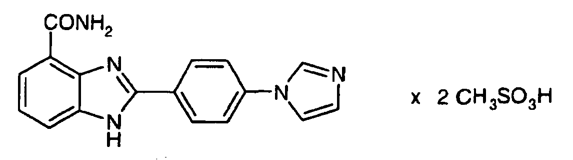

50 mg des Beispiels 4 wurden analog der Vorschrift 26a in das Bismethansulfonat überführt und lyophilisiert. Man erhielt 60 mg des Produktes.
¹H-NMR (D₆-DMSO). δ = 2,3(6H), 7,4(2H), 7,8-8,2(7H), 8,4(1H), 8,5(2H), 9,1(1H) und 9,8 (2H) ppm.

### Beispiel 29

### 2(3(3-Trifluoracetamidomethyl-pyrrol-1-yl)phenyl)-benzimidazol-4-carbonsäureamid

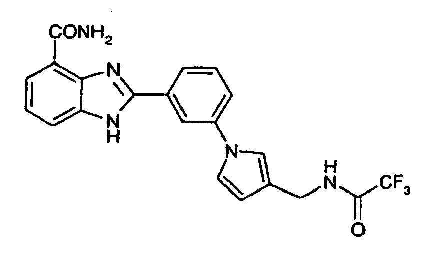

a) 2 (3-Nitrophenyl)-benzimidazol-4-carbonsäureethylester
   4,2 g (23 mMol) 2,3-Diaminobenzoesäureethylester und 3,1 ml konzentrierte Essigsäure wurden in 100 ml Methanol gelöst. Anschließend wurden 4,5 g (30 mMol) 4-Nitrobenzaldehyd, gelöst in 150 ml Methanol, innerhalb von 30Minuten zugetropft. Danach wurden 6 g (30 mMol) Kupfer-II-acetat, gelöst in 150 ml warmen Wasser, zügig zugetropft und anschließend alles für 20 Minuten unter Rückfluß gekocht. Man kühlte die Reaktionslösung auf 50oC und gab 8,3 ml konzentrierte Salzsäure zu. Danach tropfte man noch vorsichtig eine Lösung aus 8,6 g Natriumsulfid-Hydrat in 100 ml Wasser zu und rührte alles noch für 15 Minuten. Die Reaktionslösung wurde auf Eiswasser gegossen und der anfallende Niederschlag abgesaugt. Das Filtrat wurde mit wäßriger Natriumhydrogenkarbonat-Lösung alkalisch gestellt und mehrmals mit Essigester extrahiert. Die Essigester-Phase wurde abgetrennt, getrocknet und im Vakuum eingeengt. Man erhielt 6.1g des Zwischenproduktes.
b) 2(3-Nitro-phenyl)-benzimidazol-4-carbonsäurehydrazid
   Zu 6 g (19,3 mMol) der Zwischenverbindung 29a in 70 ml Ethanol wurden 4,8 g (96 mMol) Hydrazinhydrat gegeben und alles für 3 Stunden unter Rückfluß gekocht. Anschließend wurde der Reaktionsansatz auf Wasser gegossen und der entstandene Niederschlag abgesaugt. Man erhielt 4,8 g der Zwischenverbindung.
c) 2(3-Amino-phenyl)-benzimidazol-4-carbonsäureamid
   Zu 4,7 g (15,8 mMol) der Zwischenverbindung 29b in 400 ml Ethanol wurden 0,5 g Palladium-Kohle (10%ig) gegeben und alles mit Wasserstoff hydriert. Anschließend wurde der Reaktionsansatz filtriert und im Vakuum eingeengt. Der Rückstand wurde in 100 ml Dimethylformamid aufgenommen und danach mit 70 ml Wasser verdünnt. Anschließend wurden 10 g Raney-Nickel zugegeben und alles für 2 h auf 90°C erwärmt. Danach wurde filtriert und das Filtrat im Vakuum eingeengt. Der erhaltene Rückstand wurde aus Essigester/Ether kristallisiert, wonach 3,1 g des Produktes erhalten wurden.
d) 2(3(3-Triflouracetamidomethyl-pyrrol-1-yl)phenyl)-benzimidazol-4-carbonsäureamid
   2,2 g (8,7 mMol) der Zwischenverbindung 29c und 2,8 g (10,9 mMol) 2,5-Dimethoxy-3-(trifluoracet-amidomethyl)tetrahydrofuran wurden in 75 ml konzentrierter Essigsäure gegeben und für 15 Minuten unter Rückfluß erwärmt. Anschließend wurde alles im Vakuum eingeengt und der anfallende Rückstand chromatographisch an Kieselgel mit dem Fließmittel Essigester/Methanol (10/1) gereinigt. Man erhielt 2,5 g des Produktes.
   MS: m/e = 427 (M⁺).

### Beispiel 30

### 2(3(3-Aminomethyl-pyrrol-1-yl)phenyl)-benzimidazol-4-carbonsäureamid

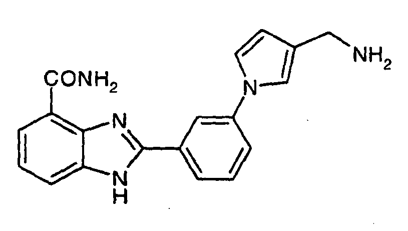

2,3 g (5,4 mMol) des Beispiels 29 wurden in 100 ml Tetrahydrofuran gelöst und mit 0,26 g (10,8 mMoll) Lithiumhydroxid, gelöst in 50 ml Wasser, versetzt. Alles wurde für 2 Stunden bei Raumtemperatur gerührt. Anschließend wurde der Ansatz durch Zugabe von verdünnter Salzsäure neutralisiert und das organische Lösungsmittel im Vakuum entfernt. Der langsam auskristallisierende Niederschlag wurde abgesaugt. Man erhielt 0,61 g des Produktes.
¹H-NMR (CF₃COOD). δ = 4,4(2H), 7,0(NH) und 7,8-8,4(11H) ppm.

### Beispiel 31

### 2(4(4-Methylpiperazin-1-yl)phenyl)-benzimidazol-4-carbonsäureamid

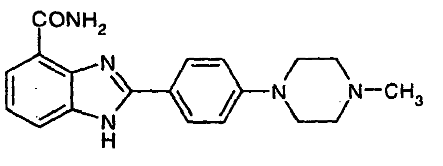

a) 4(4-Methylpiperazin-1-yl)benzaldehyd
   20 g (161 mMol) 4-Fluorbenzaldehyd, 48,4 g (483 mMol) 1-Methylpiperazin und 22,3 g (161 mMol) Kaliumkarbonat wurden in 50 ml Dimethylformamid gegeben und für 36 Stunden auf 130°C erwärmt. Anschließend wurde der Ansatz im Vakuum eingeengt. Der Rückstand wurde zwischen Essigester und 2 M Salzsäure verteilt. Die wäßrige Phase wurde abgetrennt und mit wäßriger Natriumhydrogenkarbonat-Lösung alkalisiert. Diese wäßrige Phase wurde mit Essigester extrahiert, die organische Phase abgetrennt, getrocknet und im Vakuum eingeengt. Man erhielt 48,7 g des Zwischenproduktes.
b) 2(4(4-Methylpiperazin-1-yl)phenyl)-benzimidazol-4-carbonsäureethylester
   1,5 g (8,3 mMol) 2,3-Diaminobenzoesäureethylester und 2,2 g (10,8 mMol) der Zwischenverbindung 8a wurden analog der Vorschrift 6a umgesetzt, wobei man nach einer chromatographischen Reinigung an Kieselgel 2,8 g des Produktes erhielt.
c) 2(4(4-Methylpiperazin-1-yl)phenyl)-benzimidazol-4-carbonsäurehydrazid
   1,35 g (3,7 mMol) der Zwischenverbindung 21b wurden analog der Vorschrift 6b mit Hydrazin umgesetzt, wobei 1,1 g des Produktes anfielen.
d) 2(4(4-Methylpiperazin-1-yl)phenyl)-benzimidazol-4-carbonsäureamid
   Die Zwischenverbindung wurde analog der Vorschrift 29c mit Raney-Nickel behandelt, wonach das Produkt erhalten wurde.
   ¹H-NMR (D₆-DMSO). δ = 2,25(3H), 2,6(4H), 3,2(4H), 7,0-8,1(9H) und 9,5(1H) ppm.

### Beispiel 32

### 2(3(2-Trifluoracetamidomethyl-pyrrol-1-yl)phenyl)-benzimidazol-4-carbonsäureamid

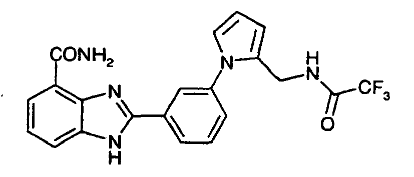

Analog Beispiel 29 wurde aus 2,3-Diaminobenzoesäureethylester, 3-Nitrobenzaldehyd und 2,5-Dimethoxy-2-(trifluoracetamidomethyl)-tetrahydrofuran die obige Verbindung hergestellt.
¹H-NMR (D₆-DMSO). δ = 4,5(2H), 6,3(2H), 7,3-8,0(6H), 9,25(1H) und 9,8(1H) ppm.

### Beispiel 33

### 2(3(3-Formyl-pyrrol-1-yl)phenyl)-benzimidazol-4-carbonsäureamid

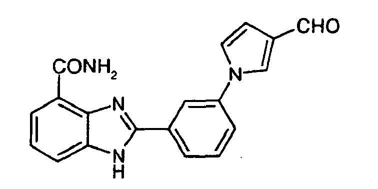

Analog Beispiel 29 wurde aus 2,3-Diaminobenzoesäureethylester, 3-Nitrobenzaldehyd und 2,5-Dimethoxy-tetrahydrofuranyl-3-carbaldehyd die obige Verbindung hergestellt.
¹H-NMR (D₆-DMSO). δ = 6,8(2H), 7,3-8,0(6H), 8,3(1H), 8,4(1H), 8,6(1H), 9,2(1H) und 9,8(1H) ppm.

### Beispiel 34

### 2(3(3(N,N-Benzyl-methylaminomethyl)-pyrrol-1-yl)phenyl)-benzimidazol-4-carbonsäureamid x 2 HCl

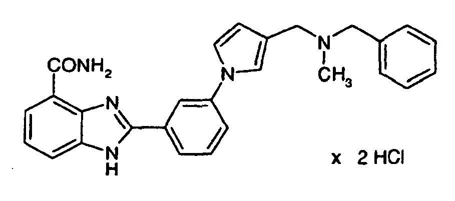

2,0 g (6,0 mMol) der Verbindung aus Beispiel 33, 0,74 g (6,0 mMol) N-Methylbenzylamin, und 0,4 ml (6,0 mMol) Eisessig wurden in 100 ml Ethanol gelöst. Bei Raumtemperatur wurden danach portionsweise 0,38 g (6,0 mMol) Natriumcyanoborhydrid zugefügt und alles bei Raumtemperatur für 16 h gerührt. Anschließend wurde der Ansatz mit wäßriger Natriumhydrogenkarbonat-Lösung verdünnt und mit Essigester extrahiert. Die organische Phase wurde abgetrennt, getrocknet und im Vakuum eingeengt. Der Rückstand wurde chromatographisch an Kieselgel (Fließmittel: Essigester/Methanol = 10/1) gereinigt. Das so erhaltene Produkt wurde in Aceton gelöst und mit isopropanolischer Chlorwasserstoff-Lösung versetzt, wobei das Produkt ausfiel und abgesaugt wurde. Man erhielt 0,98 g des Produktes.
¹H-NMR (D₆-DMSO). δ = 2,6(3H), 4,1-4,5(4H), 6,6(1H), 7,3-8,0(13H), 8,2(1H), 8,6(1H), 9,1(1H) und 10,8(1H) ppm.

### Beispiel 35

### 2(3(2-Aminomethyl-pyrrol-1-yl)phenyl)-benzimidazol-4-carbonsäureamid

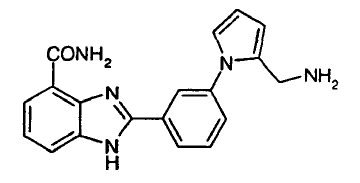

1,0 g (2,3 mMol) der Verbindung aus Beispiel 32 wurden in 100 ml Wasser gelöst und mit 0,56 g (23,4 mMol) Lithiumhydroxid, gelöst in 20 ml Wasser, versetzt. Alles wurde für 90 Minuten bei Raumtemperatur gerührt. Anschließend wurde das organische Lösungsmittel im Vakuum entfernt und die resultierende wäßrige Phase mit verdünnter Salzsäure vorsichtig neutralisiert. Der auftetende Niederschlag wurde abgesaugt. Man erhielt 0,55 g des Produktes.
¹H-NMR (D₆-DMSO). δ = 3,8(2H), 6,2(2H), 7,0(1H), 7,35(1H), 7,6-8,1(5H), 8,3(1H), 9,35(1H) und 9,5(1H) ppm.

### Beispiel 36

### 2(4(4-Methylpiperazin-1-yl)phenyl)-benzimidazol-4-carbonsäureamid x 3 HCl

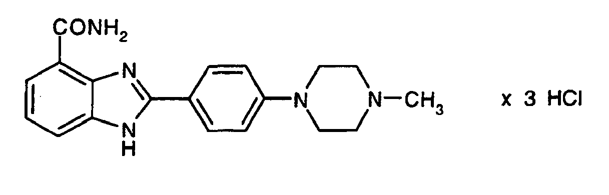

0,25 g des Produktes aus Beispiel 31 wurden in 25 ml Essigester/Tetrahydrofuran (4/1) gelöst und tropfenweise mit etherischer Chlorwasserstoffsäure versetzt. Der entstandene Niederschlag wurde mit Aceton behandelt und abgesaugt. Man erhielt 0,25 g des Produktes.
¹H-NMR (D₆-DMSO). δ = 2,75(3H), 3,1-3,4(4H), 4,0-4,4(4H), 7,25(2H), 7,5(1H), 7,9-8,1(4H), 8,3(2H), 9,0(breit) und 11,5(breit) ppm.

### Beispiel 37

### 2(4(4-tert-Butyloxypiperazin-1-yl)phenyl)-benzimidazol-4-carbonsäureamid

¹H-NMR (D₆-DMSO). δ = 1,4(9H), 3,3(4H), 3,5(4H), 7,2(1H), 7,3(1H), 7,7(1H), 7,75(1H), 7,8(1H), 8,2(2H), 9,4(1H) und 12,5 ppm.

### Beispiel 38

### 2(4(Piperazin-1-yl)phenyl)-benzimidazol-4 -carbonsäureamid x 2 HCl

¹H-NMR (D₆-DMSO). δ = 3,3(4H), ca. 3,7(4H), 7,3(2H), 7,6(1H), 7,9-8,0(3H), 8,3(2H), 8,7(1H) und 9,5(breit) ppm.

### Beispiel 39

### 2(3(2(Aminomethyl)-pyrrol-1-yl)phenyl)-benzimidazol-4-carbonsäureamid x 2 HCl

¹H-NMR (D₂O). δ = 4,25(2H), 6,4(1H), 6,6(1H), 7,1(1H), 7,4(1H), 7,6(1H), 7,7-7,8(3H), 7,9(1H) und 8,0(1H) ppm.

### Beispiel 40

### 2(4(3-Formylpyrrol-1-yl)phenyl)-benzimidazol-4-carbonsäureamid

¹H-NMR (D₆-DMSO). δ = 6,7(1H), 7,3(1H), 7,7-8,0(7H), 8,4(2H), 9,4(1H), 9,8(1H) und 13,5(breit) ppm.

### Beispiel 41

### 2 (4 (3-(N,N-Benzylmethylaminomethyl)-pyrrol-1-yl)phenyl)-benzimidazol-4-carbonsäureamid x 2 HCl

MS: m/e = 435 (M⁺).

### Beispiel 42

### 2 (4 (3-(N,N-Diethylaminomethyl)-pyrrol-1-yl)phenyl)-benzimidazol-4-carbonsäureamid x 2 HCl

¹H-NMR (D₆-DMSO). δ = 1,3(6H), 3,1(4H), 4,2(2H), 6,6(1H), 7,5(1H), 7,75(1H), 7,8-8,0(6H), 8,5(2H), 9,1(1H) und 10,4(1H) ppm.

### Beispiel 43

### 2 (4(3-(4-Methylpiperazin-1-ylmethyl)-pyrrol-1-yl)phenyl)-benzimidazol-4-carbonsäureamid

¹H-NMR (D₆-DMSO). δ = 2,1(3H), 2,2-2,5(8H), 3,35(2H), 6,2(1H), 7,3-8,0(7H), 8,3(2H) und 9,4(breit) ppm.

### Beispiel 44

### 2(4(3-(4-Benzylpiperazin-1-ylmethyl)-pyrrol-1-yl)phenyl)-benzimidazol-4-carbonsäureamid

¹H-NMR (D₆-DMSO). δ = 2,2-2,6(8H), 3,4(2H), 3,5(2H), 6,2(1H), 7,2-8,0(13H), 8,3(2H), 9,4(1H) und 13,4(breit) ppm.

### Beispiel 45

### 2 (4(3-(Piperidin-1-ylmethyl)-pyrrol-1-yl)phenyl)-benzimidazol-4-carbonsäureamid

¹H-NMR (D₆-DMSO). δ = 1,3-1,6(6H), 2,3(4H), 3,3(2H), 6,2(1H), 7,3-8,0(8H), 8,3(2H) und 9,4(breit) ppm.

### Beispiel 46

### 2(4(4-Benzylpiperazin-1-yl)phenyl)-benzimidazol-4-carbonsäureamid 3 x HCl

¹H-NMR (D₆-DMSO). δ = 3,2(4H), 4,2(4H), 4,5(2H), 7,2(2H), 7,4-8,0(9H), 8,2(2H), 9,0(1H) und 11,8(breit) ppm.

### Beispiel 47

### 2(4(4-Cyclohexylpiperazin-1-yl)phenyl)-benzimidazol-4-carbonsäureamid

¹H-NMR (D₆-DMSO). δ = 1,1-1,9(10H), 2,7(4H), 3,2(4H), 4,1(1H), 7,1(2H), 7,25(1H), 7,7(2H), 7,8(1H), 8,0(2H), 9,4(1H) und ca. 13(breit) ppm.

### Beispiel 48

### 2(4(4-Ethylpiperazin-1-yl)phenyl)-benzimidazol-4-carbonsäureamid

¹H-NMR (D₆-DMSO). δ = 1,0(3H), 2,4(2H), 2,5(4H), 3,2(4H), 7,0-7,3(3H), 7,6-7,9(2H), 8,0(2H), 9,4(1H) und ca. 13(breit) ppm.

### Beispiel 49

### 2(4(4-n-Butylpiperazin-1-yl)phenyl)-benzimidazol-4-carbonsäureamid

¹H-NMR (D₆-DMSO). δ = 0,9(3H), 1,2-1,6(4H), 2,3(2H), 3,2-3,5(8H), 7,1(2H), 7,3(1H), 7,6-7,9(3H), 8,1(2H), 9,5(1H) und 13(breit)ppm.

### Beispiel 50

### 2(4(4-Diphenylmethylpiperazin-1-yl)phenyl)-benzimidazol-4-carbonsäureamid

¹H-NMR (D₆-DMSO). δ = 2,5(4H), 3,2(4H), 4,3(1H), 7,0-7,9(16H), 8,1(2H), 9,4(1H) und ca. 13(breit) ppm.

### Beispiel 51

### 2(2-Methyl-4-piperazin-1-yl-phenyl)-benzimidazol-4-carbonsäureamid 3 x HCl

MS: m/e = 335(M⁺).

### Beispiel 52

### 2(3-Piperazin-1-yl-phenyl)-benzimidazol-4-carbonsäureamid 3 x HCl

¹H-NMR (D₆-DMSO). δ = 3,2(4H), 3,6(2H), 7,2-7,6(3H), 7,7-8,0(4H), 8,9(breit) und 9,5(breit) ppm.

### Beispiel 53

### 2 (4 (4-Isopropylpiperazin-1-yl)phenyl)-benzimidazol-4-carbonsäureamid

¹H-NMR (D₆-DMSO). δ = 1,0(6H), 2,7(4H), 2,8(1H), 3,3(4H), 7,1(2H), 7,2(1H), 7,5-7,9(3H), 8,05(2H), 9,4(1H) und 13 (breit) ppm.

### Beispiel 54

### 2(4(4-tert.Butyloxycarbonylhomopiperazin-1-yl)phenyl)-benzimidazol-4-carbonsäureamid

¹H-NMR (D₆-DMSO). δ = 1,1-1,3(9H), 1,9(2H), 3,1-3,9(8H), 6,9(2H), 7,2(1H), 7,7-7,9(3H), 8,0(2H), 9,5(1H) und ca. 13(breit) ppm.

### Beispiel 55

### 2(4(Homopiperazin-1-yl)phenyl)-benzimidazol-4-carbonsäureamid

¹H-NMR (D₆-DMSO). δ = 2,1(2H), 3,1(2H), 3,2(2H), 3,7(2H), 3,9(2H), 7,0(2H), 7,5(1H), 7,8-8,0(3H), 8,2(2H), 8,7(breit) und 9,3(breit) ppm.

### Beispiel 56

### 2(4(4-(Piperidin-1-yl)piperidin-1-yl)phenyl)-benzimidazol-4-carbonsäureamid

¹H-NMR (D₆-DMSO). δ = 1,7-1,9(8H), 2,2(2H), 2,8-2,9(3H), 3,3(4H), 4,1(2H), 7,1(2H), 7,3(1H), 7,7(1H), 7,75(1H), 7,8(1H), 8,1(2H), 9,4(1H) und 13,2(breit) ppm.

### Beispiel 57

### 2(4(3-Amino-pyrroldin-1-yl)phenyl)-benzimidazol-4-carbonsäureamid x 2 HCl

MS: m/e = 321 (M⁺).

### Beispiel 58

### 2(4(4-Benzyl-homopiperazin-1-yl)phenyl)-benzimidazol-4-carbonsäureamid

### Beispiel 59

### 2(4(4-Methyl-homopiperazin-1-yl)phenyl)-benzimidazol-4-carbonsäureamid

### Beispiel 60

### 2(4(4-Ethyl-homopiperazin-1-yl)phenyl)-benzimidazol-4-carbonsäureamid

### Beispiel 61

### 2(4(4-Isopropyl-homopiperazin-1-yl)phenyl)-benzimidazol-4-carbonsäureamid

### Beispiel 62

### 2 (4 (4-Butyl-homopiperazin-1-yl)phenyl)-benzimidazol-4-carbonsäureamid

### Beispiel 63

### Synthese von 2-Phenylbenzimidazol-4-carboxamid

a) 2,3-Diamino-benzoesäureamid x 2 Hydrochlorid
   Eine Lösung von 200 g (1,11 mol) 2,3-Diaminobenzoesäureethylester in 1500 ml 1-Butanol wurde bei Raumtemperatur vorsichtig mit 400 ml Hydrazinhydrat versetzt. Die Mischung wurde 15 Stunden auf 100°C erwärmt. Anschließend wurde der Ansatz auf ein Drittel des Volumens eingeengt. Diese Lösung wurde langsam zu einer Suspension von ca. 200 g Raney-Nickel in 500 ml Wasser und 1000 ml Dimethylformamid getropft. Die Mischung wurde 2 Stunden auf 100°C erwärmt. Nach Abkühlen auf 10°C wurde der Katalysator abgetrennt und das Filtrat im Vakuum eingeengt. Das so erhaltene Öl wurde in 500 ml Methanol gelöst und mit Diethylether versetzt. Der Niederschlag wurde abgetrennt und das Filtrat erneut eingeengt. Eine Lösung des erhaltenen Öls in Methanol wurde unter Rückfluß mit Chlorwasserstoff/iso-Propanol versetzt. Der beim Abkühlen ausfallende Niederschlag wurde abgesaugt, mit Diethylether aufgeschlämmt und erneut abgesaugt. Man erhielt 172,2 g des Produktes.
b) 2-Phenylbenzimidazol-4-carboxamid
   Zu einer Lösung von 0,84 g (15 mmol) Kaliumhydroxidpulver in 100 ml Ethanol wurden bei Raumtemperatur 1,68 g (7,5 mmol) des Produktes aus 1b zugegeben. Nach 5 Minuten wurden 1,35 g (22,5 mmol) Eisessig zugegeben, und innerhalb von 30 Minuten wurde eine Lösung von 1 g (9,38 mmol) Benzaldehyd in 20 ml Ethanol zugetropft. Anschließend wurde eine Lösung von 2,59 g (12,97 mmol) Kupfer-II-acetat in 20 ml dest. Wasser zügig zugetropft. Die Mischung wurde 2 Stunden unter Rückfluß erhitzt. Der Ansatz wurde auf Wasser gegeben, mit Konzentrierter Ammoniaklösung alkalisch gestellt und mit Ethylacetat extrahiert. Die organische Phase wurde mit Wasser gewaschen und unter Zusatz von Aktivkohle über Magnesiumsulfat getrocknet und im Vakuum eingeengt. Der harzige Rückstand wurde mit Diethylether verrieben, das abgetrennte Kristallisat wurde mit Diethylether gewaschen und im Vakuum getrocknet.
   Es wurden 1,5 g des Produktes erhalten.

## Patentansprüche

1. Verbindungen der Formel I oder II 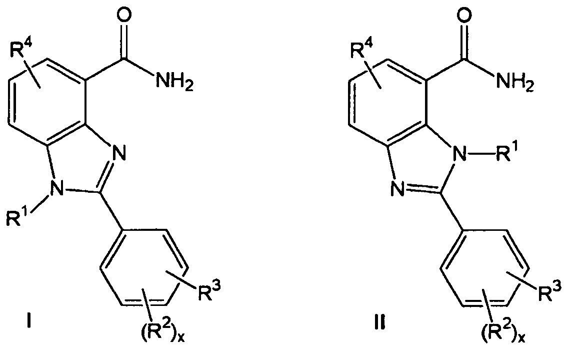 worin
R¹ Wasserstoff, verzweigtes und unverzweigtes C₁-C₆-Alkyl, wobei ein C-Atom des Alkyl-Restes noch OR¹¹ oder eine Gruppe R⁵ tragen kann, wobei R¹¹ Wasserstoff oder C₁-C₄-Alkyl bedeutet, und
R² Wasserstoff, Chlor, Brom, Jod, Fluor, CF₃, Nitro, NHCOR²¹, NR²²R²³, OH, O-C₁-C₄-Alkyl, O-C₁-C₄-Alkyl-Phenyl, NH₂, Phenyl, wobei die Phenyl-Ringe noch mit maximal zwei Resten R²⁴ substituiert sein können, und R²¹ und R²² unabhängig voneinander Wasserstoff oder C₁-C₄-Alkyl bedeuten und R²³ Wasserstoff, C₁-C₄-Alkyl oder Phenyl bedeuten, und R²⁴ OH, C₁-C₆-Alkyl, O-C₁-C₄-Alkyl, Chlor, Brom, Jod, Fluor, CF₃, Nitro, NH₂, und
x 0, 1 und 2 sein kann und
R³ ist B
R⁴ Wasserstoff, Chlor, Fluor, Brom, Jod, verzweigtes und unverzweigtes C₁-C₆-Alkyl, OH, Nitro, CF₃, CN, NR⁴¹R⁴², NH-CO-R⁴³, O-C₁-C₄-Alkyl, wobei R⁴¹ und R⁴² unabhängig voneinander Wasserstoff oder C₁-C₄-Alkyl bedeuten und
R⁴³ Wasserstoff, C₁-C₄-Alkyl, C₁-C₄-Alkyl-Phenyl oder Phenyl bedeuten, und
B 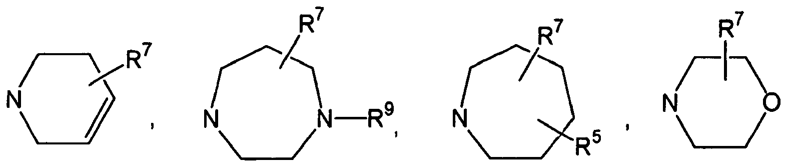 sein kann und
R⁵ Wasserstoff, C₁-C₆-Alkyl, NR⁷R⁹ und 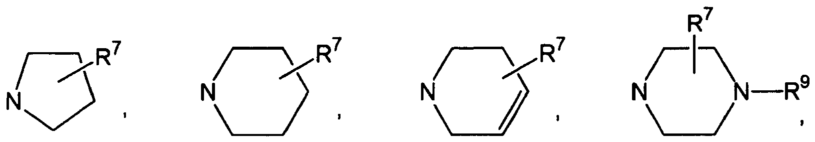 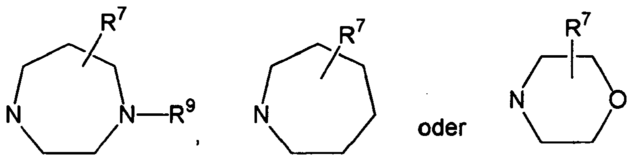 bedeuten kann und
R⁷ Wasserstoff, C₁-C₆-Alkyl, C₁-C₄-Alkyl-Phenyl, Phenyl, wobei die Ringe noch mit bis zu zwei Resten R⁷¹ substituiert sein können, und
R⁷¹ OH, C₁-C₆-Alkyl, O-C₁-C₄-Alkyl, Chlor, Brom, Jod, Fluor, CF₃, Nitro, NH₂, und
R⁹ Wasserstoff, COCH₃, CO-O-C₁-C₄-Alkyl, COCF₃, verzweigtes und unverzweigtes C₁-C₆-Alkyl, wobei ein oder zwei Wasserstoffe des C₁-C₆-Alkylrests durch jeweils einen der folgenden Reste substituiert sein kann: OH, O-C₁-C₄-Alkyl und Phenyl und der Phenyl-Ring noch einen oder zwei der folgenden Reste tragen kann: Jod, Chlor, Brom, Fluor, verzweigtes und unverzweigtes C₁-C₆-Alkyl, Nitro, Amino, C₁-C₄-Alkylamino, C₁-C₄-Dialkylamino, OH, O-C₁-C₄-Alkyl, CN, CF₃, SO₂-C₁-C₄-Alkyl, bedeuten kann, und
sowie ihre tautomeren Formen, möglichen enantiomeren und diastereomeren Formen und pharmakologisch verträglichen Salze.

2. Verbindungen nach Anspruch 1, wobei R² in 3-Position und R³ in 4-Position oder R² in 4-Position und R³ in 3-Position zum Benzimidazolring steht.

3. Verbindungen nach einem der Ansprüche 1 oder 2, wobei R¹ und R⁴ Wasserstoff bedeuten.

4. Verbindungen nach einem der Ansprüche 1 bis 3, wobei R² Wasserstoff, verzweigtes oder unverzweigtes C₁-C₆-Alkyl, Nitro, CN, NH₂, O-C₁-C₄-Alkyl bedeutet.

5. Verbindungen nach einem der Ansprüche 1 bis 4, wobei R⁵ einen 6-gliedrigen Ring bedeutet.

6. Arzneimittel, enthaltend neben den üblichen Träger und Hilfsstoffen eine Verbindung nach einem der Ansprüche 1 bis 5.

7. Verwendung von Verbindungen der Formel I nach einem der Ansprüche 1 bis 6 zur Herstellung von Arzneimitteln zur Behandlung von Krankheiten, bei denen pathologisch erhöhte Aktivitäten von PARP auftreten.

8. Verwendung von Verbindungen der Formel I nach Anspruch 7 zur Herstellung von Arzneimitteln zur Behandlung von neuro-degenerativen Krankheiten und neuronalen Schädigungen.

9. Verwendung nach Anspruch 7 zur Behandlung von solchen neuro-degenerativen Krankheiten und neuronalen Schädigungen, die durch Ischämie, Trauma oder Massenblutungen ausgelost werden.

10. Verwendung nach Anspruch 7 zur Behandlung des Schlaganfalls und des Schädel-Himtraumas.

11. Verwendung nach Anspruch 7 zur Behandlung der Alzheimerschen Krankheit der Parkinsonsche Krankheit und der Huntington-Krankheit.

12. Verwendung von Verbindungen der Formel I nach Anspruch 7 zur Herstellung von Arzneimitteln zur Behandlung oder Prophylaxe von Schädigungen durch Ischämien.

13. Verwendung von Verbindungen der Formel I nach Anspruch 7 zur Herstellung von Arzneimitteln zur Behandlung von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen.

14. Verwendung von Verbindungen der Formel I nach Anspruch 7 zur Herstellung von Arzneimitteln zur Behandlung von Schädigungen der Nieren nach renalen Ischämien, Schädigungen, die durch medikamentöse Therapie verursacht werden, wie zum Beispiel während der Cyclosporin-Therapie, und zur Behandlung während und nach Nierentransplantationen.

15. Verwendung von Verbindungen der Formel I nach Anspruch 7 zur Herstellung von Arzneimitteln zur Behandlung von Schädigungen des Herzens nach cardialen Ischämien.

16. Verwendung von Verbindungen der Formel I nach Anspruch 7 zur Herstellung von Arzneimitteln zur Behandlung von Mikroinfarkten wie zum Beispiel während und nach Herzklappenersatz, Aneurysmenresektionen und Herztransplantationen.

17. Verwendung von Verbindungen der Formel I nach Anspruch 7 zur Herstellung von Arzneimitteln zur Behandlung bei einer Revasculariation kritischer verengter Koronararterien wie zum Beispiel bei PTCA und Bypass-Operationen oder kritisch verengter peripherer Arterien, insbesondere Beinarterien.

18. Verwendung von Verbindungen der Formel I nach Anspruch 7 zur Herstellung von Arzneimitteln zur Behandlung des akuten Myocardinfarktes und von Schädigungen während und nach dessen medikamentöser oder mechanischer Lyse.

19. Verwendung von Verbindungen der Formel I nach Anspruch 7 zur Herstellung von Arzneimitteln zur Behandlung von Tumoren und deren Metastasierung.

20. Verwendung von Verbindungen der Formel I nach Anspruch 7 zur Herstellung von Arzneimitteln zur Behandlung von Sepsis des Multiorganversagens wie zum Beispiel während des septischen Schocks und des "acute respiratory distress-syndroms".

21. Verwendung von Verbindungen der Formel I nach Anspruch 7 zur Herstellung von Arzneimitteln zur Behandlung von immunologischen Krankheiten wie Entzündungen und rheumatische Erkrankungen, wie zum Beispiel rheumatoide Arthritis.

22. Verwendung von Verbindungen der Formel I nach Anspruch 7 zur Herstellung von Arzneimitteln zur Behandlung von Diabetes mellitus.

## Claims

1. Compounds of the formula I or II 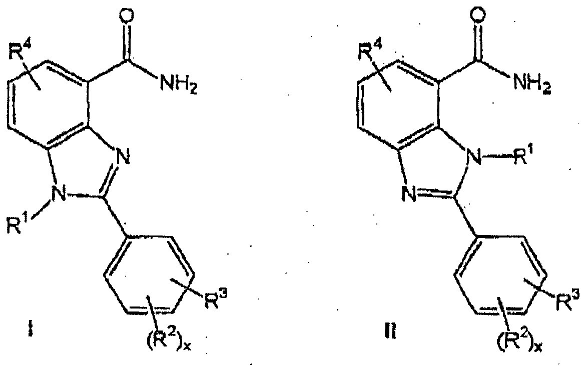 wherein
R¹ denotes hydrogen, branched and unbranched C₁-C₆ alkyl, in which a C atom of the alkyl radical may also carry OR¹¹ or a group R⁵, in which R¹¹ denotes hydrogen or C₁-C₄ alkyl, and
R² denotes hydrogen, chlorine, bromine, iodine, fluorine, CF₃, nitro, NHCOR²¹, NR²²R²³, OH, O-C₁-C₄ alkyl, O-C₁-C₄ alkylphenyl, NH₂, phenyl, in which the phenyl rings may also be substituted with at most two radicals R²⁴, and R²¹ and R²² independently of one another denote hydrogen or C₁-C₄ alkyl and R²³ denotes hydrogen, C₁-C₄ alkyl or phenyl, and R²⁴ denotes OH, C₁-C₆ alkyl, O-C₁-C₄ alkyl, chlorine, bromine, iodine, fluorine, CF₃, nitro, NH₂ and
x may be 0, 1 and 2, and
R³ is B
R⁴ denotes hydrogen, chlorine, fluorine, bromine, iodine, branched and unbranched C₁-C₆ alkyl, OH, nitro, CF₃, CN, NR⁴¹R⁴², NH-CO-R⁴³, O-C₁-C₄ alkyl, in which R⁴¹ and R⁴² independently of one another denote hydrogen or C₁-C₄ alkyl, and
R⁴³ denotes hydrogen, C₁-C₄ alkyl, C₁-C₄ alkylphenyl or phenyl, and
B may be 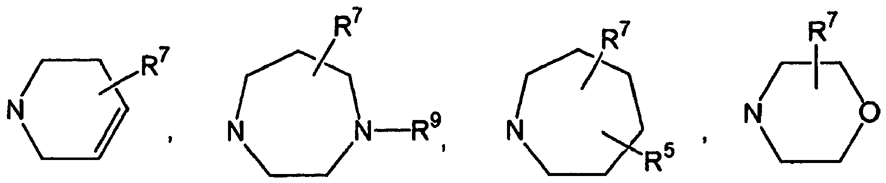 and
R⁵ may denote hydrogen, C₁-C₆ alkyl, NR⁷R⁹ and 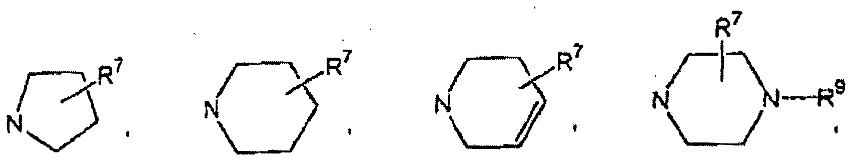 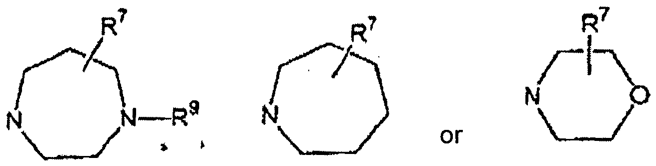 and
R⁷ denotes hydrogen, C₁-C₆ alkyl, C₁-C₄ alkylphenyl, phenyl, in which the rings may also be substituted with up to two radicals R⁷¹, and
R⁷¹ denotes OH, C₁-C₆ alkyl, O-C₁-C₄ alkyl, chlorine, bromine, iodine, fluorine, CF₃, nitro, NH₂, and
R⁹ denotes hydrogen, COCH₃, CO-O-C₁-C₄ alkyl, COCF₃, branched and unbranched C₁-C₆ alkyl, in which one or two hydrogens of the C₁-C₆ alkyl radical may be substituted by in each case one of the following radicals: OH, O-C₁-C₄ alkyl and phenyl and the phenyl ring may also carry one or two of the following radicals: iodine, chlorine, bromine, fluorine, branched and unbranched C₁-C₆ alkyl, nitro, amino, C₁-C₄ alkylamino, C₁-C₄ dialkylamino, OH, O-C₁-C₄ alkyl, CN, CF₃, SO₂-C₁-C₄ alkyl,
as well as their tautomeric forms, possible enantiomeric and diastereomeric forms, and pharmacologically compatible salts.

2. Compounds according to claim 1, in which R² is in the 3-position and R³ is in the 4-position or R² is in the 4-position and R³ is in the 3-position to the benzimidazole ring.

3. Compounds according to one of claims 1 or 2, in which R¹ and R⁴ denote hydrogen.

4. Compounds according to one of claims 1 to 3, in which R² denotes hydrogen, branched or unbranched C₁-C₆ alkyl, nitro, CN, NH₂, O-C₁-C₄ alkyl.

5. Compounds according to one of claims 1 to 4, in which R⁵ denotes a 6-membered ring.

6. Medicament containing a compound according to one of claims 1 to 5 in addition to the conventional carriers and auxiliary substances.

7. Use of compounds of the formula I according to one of claims 1 to 6 for the production of medicaments for treating diseases in which pathologically raised activities of PARP occur.

8. Use of compounds of the formula I according to claim 7 for the production of medicaments for treating neurodegenerative diseases and neuronal damage.

9. Use according to claim 7 for the treatment of such neurodegenerative diseases and neuronal damage that are triggered by ischaemia, trauma or massive bleeding.

10. Use according to claim 7 for the treatment of strokes and craniocerebral trauma.

11. Use according to claim 7 for the treatment of Alzheimer's disease, Parkinson's disease and Huntington's disease.

12. Use of compounds of the formula I according to claim 7 for the production of medicaments for the treatment or prophylaxis of damage due to ischaemia.

13. Use of compounds of the formula I according to claim 7 for the production of medicaments for treating epilepsy, in particular generalised epileptic attacks, such as for example petit mal and tonic-clonic attacks and partial epileptic attacks such as temporal lobe and complex-partial attacks.

14. Use of compounds of the formula I according to claim 7 for the production of medicaments for treating kidney damage after renal ischaemia, damage caused by medical therapy, such as for example during cyclosporin therapy, and for treatment during and after kidney transplants.

15. Use of compounds of the formula I according to claim 7 for the production of medicaments for treating cardiac damage after cardiac ischaemia.

16. Use of compounds of the formula I according to claim 7 for the production of medicaments for treating microinfarcts, such as, for example, during and after heart-valve replacement, aneurysm resections and heart transplants.

17. Use of compounds of the formula I according to claim 7 for the production of medicaments for treatment involving a revascularisation of critically constricted coronary arteries, such as, for example, in PTCA and bypass operations, or critically constricted peripheral arteries, in particular leg arteries.

18. Use of compounds of the formula I according to claim 7 for the production of medicaments for the treatment of acute myocardial infarction and damage during and after the medical or mechanical lysis thereof.

19. Use of compounds of the formula I according to claim 7 for the production of medicaments for treating tumours and metastases thereof.

20. Use of compounds of the formula I according to claim 7 for the production of medicaments for treating sepsis in multi-organ failure, such as, for example, during septic shock and acute respiratory distress syndrome.

21. Use of compounds of the formula I according to claim 7 for the production of medicaments for treating immunological diseases such as inflammatory and rheumatic diseases, such as, for example, rheumatoid arthritis.

22. Use of compounds of the formula I according to claim 7 for the production of medicaments for treating diabetes mellitus.

## Revendications

1. Composés selon la formule I ou II 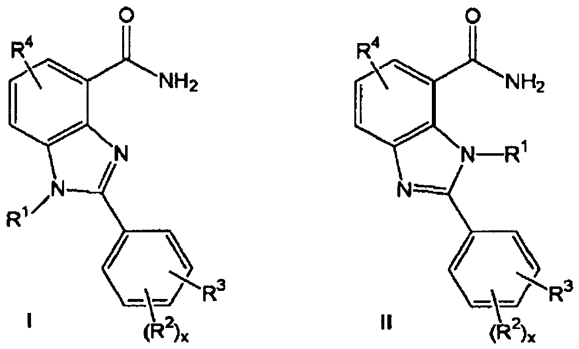 dans lesquels
R¹ désigne un groupe hydrogène, un groupe C₁-C₆-alkyle ramifié ou non ramifié dans lequel un atome de carbone du groupe fonctionnel alkyle peut encore porter un groupe OR¹¹ ou R⁵ , où R¹¹ désigne un groupe hydrogène ou C₁-C₄-alkyle, et
R² désigne un groupe hydrogène, chlore, brome, iode, fluor, CF₃, nitro, NHCOR²¹, NR²²R²³, OH, O-C₁-C₄-alkyle, O-C₁-C₄-alkyl-phényle, NH₂, phényle, dans lesquels les cycles phényle peuvent aussi être substitués par jusqu'à deux groupes fonctionnels R²⁴, R²¹ et R²² désignent indépendamment l'un de l'autre un groupe hydrogène ou C₁-C₄-alkyle, R²³ désigne un groupe hydrogène, C₁-C₄-alkyle ou phényle, et R²⁴ désigne un groupe OH, C₁-C₆-alkyle, O-C₁-C₄-alkyle, chlore, brome, iode, fluor, CF₃, nitro ou NH₂,
x peut valoir 0, 1 ou 2,
R³ est B,
R⁴ désigne un groupe hydrogène, chlore, fluor, brome, iode, C₁-C₆-alkyle ramifié ou non ramifié, OH, nitro, CF₃, CN, NR⁴¹R⁴² NH-CO-R⁴³, O-C₁-C₄-alkyle, dans lesquels R⁴¹ et R⁴² désignent indépendamment l'un de l'autre un groupe hydrogène ou C₁-C₄-alkyle,
R⁴³ désigne un groupe hydrogène, C₁-C₄-alkyle, C₁-C₄-alkyle-phényle ou phényle, et
B peut être 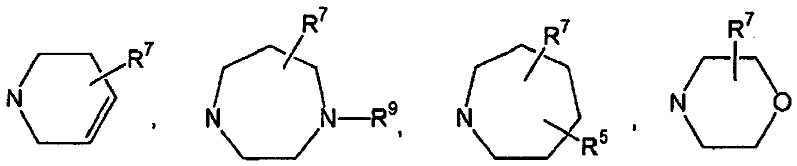 et
R⁵ désigne un groupe hydrogène, C₁-C₆-alkyle ou NR⁷R⁹ et peut désigner 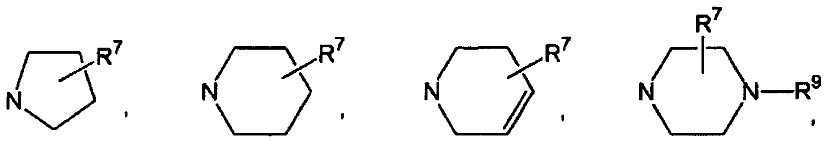 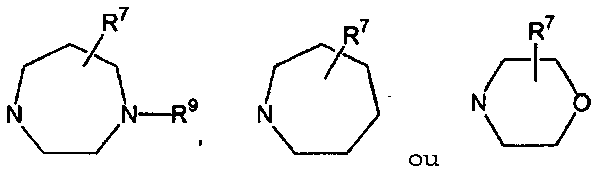 et
R⁷ désigne un groupe hydrogène, C₁-C₆-alkyle, C₁-C₄-alkyle-phényle ou phényle, dans lesquels les cycles peuvent aussi être substitués par jusqu'à deux groupes fonctionnels R⁷¹,
R⁷¹ désigne un groupe OH, C₁-C₆-alkyle, O-C₁-C₄-alkyle, chlore, brome, iode, fluor, CF₃, nitro ou NH₂, et
R⁹ désigne un groupe hydrogène, COCH₃, CO-O-C₁-C₄-alkyle, COCF₃, C₁-C₆-alkyle ramifié ou non ramifié, dans lesquels un ou deux hydrogènes du groupe fonctionnel C₁-C₆-alkyle peuvent être substitués chacun par un des groupes fonctionnels suivants : OH, O-C₁-C₄-alkyle ou phényle, et le cycle phényle peut encore comporter un ou deux des groupes fonctionnels suivants : iode, chlore, brome, fluor, C₁-C₆-alkyle ramifié ou non ramifié, nitro, amino, C₁-C₄-alkylamino, C₁-C₄-dialkylamino, OH, O-C₁-C₄-alkyle, CN, CF₃, SO₂-C₁-C₄-alkyle,
ainsi que leurs formes tautomères, éventuellement énantiomères et diastéréoisomères, ainsi que leurs sels compatibles pour un usage pharmaceutique.

2. Composés selon la revendication 1, dans lesquels R² se trouve en position 3 et R³ en position 4, ou R² en position 4 et R³ en position 3, par rapport au cycle benzimidazole.

3. Composés selon l'une des revendications 1 ou 2, dans lesquels R¹ et R⁴ désignent un groupe hydrogène.

4. Composés selon l'une des revendications 1 à 3, dans lesquels R² désigne un groupe hydrogène, C₁-C₆-alkyle ramifié ou non ramifié, nitro, CN, NH₂ ou O-C₁-C₄-alkyle.

5. Composés selon l'une des revendications 1 à 4, dans lesquels R⁵ désigne un cycle à 6 éléments.

6. Médicament comprenant, outre les excipients et additifs habituels, un composé selon l'une des revendications 1 à 5.

7. Utilisation de composés de la formule I selon l'une des revendications 1 à 6 pour la fabrication de médicaments destinés au traitement de maladies dans lesquelles apparaît une activité pathologique accrue des poly(ADP-ribose) polymérases, soit PARP.

8. Utilisation de composés de la formule I selon la revendication 7 pour la fabrication de médicaments destinés au traitement de maladies neurodégénératives et de lésions neuronales.

9. Utilisation selon la revendication 7 pour le traitement de maladies neurodégénératives et de lésions neuronales provoquées par une ischémie, un traumatisme ou une hémorragie massive.

10. Utilisation selon la revendication 7 pour le traitement d'un accident vasculaire cérébral ou d'un traumatisme cranio-cérébral.

11. Utilisation selon la revendication 7 pour le traitement de la maladie d'Alzheimer, de la maladie de Parkinson ou de la maladie de Huntington.

12. Utilisation de composés de la formule I selon la revendication 7 pour la fabrication de médicaments destinés au traitement ou à la prophylaxie de lésions par ischémie.

13. Utilisation de composés de la formule I selon la revendication 7 pour la fabrication de médicaments destinés au traitement de l'épilepsie, en particulier de crises épileptiques généralisées, par exemple le petit mal épileptique et les crises tonico-cloniques, de crises épileptiques partielles, par exemple du lobe temporal, et de crises partielles complexes.

14. Utilisation de composés de la formule I selon la revendication 7 pour la fabrication de médicaments destinés au traitement de lésions des reins après une ischémie ou une lésion rénale provoquée par une thérapie médicamenteuse, par exemple lors d'une thérapie à base de ciclosporine, et pour un traitement pendant et après une transplantation rénale.

15. Utilisation de composés de la formule I selon la revendication 7 pour la fabrication de médicaments destinés au traitement de lésions cérébrales après une ischémie cardiaque.

16. Utilisation de composés de la formule I selon la revendication 7 pour la fabrication de médicaments destinés au traitement de micro-infarctus, par exemple pendant et après un remplacement de valve cardiaque, une résection d'anévrisme ou une transplantation cardiaque.

17. Utilisation de composés de la formule I selon la revendication 7 pour la fabrication de médicaments destinés à un traitement de revascularisation d'artères coronaires à rétrécissement critique, par exemple lors d'interventions d'angioplastie coronaire et de bypass ou lors d'une artériopathie oblitérante, en particulier des membres inférieurs.

18. Utilisation de composés de la formule I selon la revendication 7 pour la fabrication de médicaments destinés au traitement de lésions et d'infarctus aigus du myocarde pendant et après leur lyse médicamenteuse ou mécanique.

19. Utilisation de composés de la formule I selon la revendication 7 pour la fabrication de médicaments destinés au traitement de tumeurs et de leurs métastases.

20. Utilisation de composés de la formule I selon la revendication 7 pour la fabrication de médicaments destinés au traitement d'un sepsis de défaillance multiviscérale, par exemple lors d'un choc septique ou d'un syndrome de détresse respiratoire aiguë.

21. Utilisation de composés de la formule I selon la revendication 7 pour la fabrication de médicaments destinés au traitement de maladies immunologiques telles que les inflammations et les affections rhumatiques, par exemple pour la polyarthrite rhumatoïde.

22. Utilisation de composés de la formule I selon la revendication 7 pour la fabrication de médicaments destinés au traitement du diabète sucré.
